(19) Europäisches Patentamt
European Patent Office
Office européen des brevets

(11) Publication number: 0 367 408 A2

(12) # EUROPEAN PATENT APPLICATION

(21) Application number: 89310111.3

(22) Date of filing: 03.10.89

(51) Int. Cl.5: C07C 45/53 , C07C 49/08

A request for addition of table VI has been filed pursuant to Rule 88 EPC. A decision on the request will be taken during the proceedings before the Examining Division (Guidelines for Examination in the EPO, A-V, 2.2).

(30) Priority: 24.10.88 US 261817
24.10.88 US 261818
24.10.88 US 261819
17.03.89 US 324118

(43) Date of publication of application: 09.05.90 Bulletin 90/19

(84) Designated Contracting States: DE FR GB IT

(71) Applicant: TEXACO DEVELOPMENT CORPORATION
2000 Westchester Avenue
White Plains, New York 10650(US)

(72) Inventor: Knifton, John Frederick
10900 Catskill Trail
Austin Texas 78750(US)
Inventor: Sanderson, John Ronald
15290 Faubion Trail
Leander Texas 78761(US)
Inventor: Grice, Neal John
2403 Akron Cove
Austin Texas 78723(US)

(74) Representative: Brock, Peter William et al
URQUHART-DYKES & LORD 91 Wimpole Street
London W1M 8AH(GB)

(54) **Method for production of phenol/acetone from cumene hydroperoxide.**

(57) Cumene hydroperoxide can be decomposed into phenol and acetone under mild conditions (a temperatur of 20 to 150° C and a pressure of 0.1 to 7 MPa) by means of a heterogeneous catalyst selected from:
a heteropoly acid on an inert support,
an ion exchange resin with a sulfonic acid functionality,
an acidic smectite clay,
a fluorine-containing acid on an inert support, and montmorillonite clay modified by a heteropoly acid or b titanium, zirconium or aluminium.



Xerox Copy Centre

## METHOD FOR PRODUCTION OF PHENOL/ACETONE FROM CUMENE HYDROPEROXIDE

This invention relates to novel methods for the decomposition of organic hydroperoxides, and mor particularly this invention relates to a method for producing phenol and acetone by decomposition ( cumene hydroperoxide over certain heterogeneous catalysts. The invention is particularly advantageous i that there is quantitative conversion of cumene hydroperoxide under mild conditions. The catalysts are ve attractive because by-products are produced in much smaller percentages than with standard acid catalysi.

It is known that cumene can be oxidized to cumene hydroperoxide, and that cumene hydroperoxide ca be decomposed by various means to provide phenol and acetone.

US-A-2889368 discloses a process for the decomposition of various organic hydroperoxides, fc example, cumene hydroperoxide. The cumene hydroperoxide is decomposed to phenol and acetone in th presence of a 10 to 70% aqueous sulphuric acid solution at a temperature between 50 and 100°C, th yields amounting to 80 to 90%.

The disadvantages of using soluble strong acid catalysts for this purpose include a) the need for a efficient means of separating the phenol/acetone products from the aid or spent acid catalyst, b) the need t neutralize these acids, e.g. with caustic soda, c) the disposal of salts generated as a result of th neutralization, and d) the difficulty in obtaining > 99.9% purity phenol from such a process if there is an entrainment or contamination of the crude phenol/acetone product by the acid catalyst.

US-A-2715145 concerns a process for increasing the yield of phenol by decomposing the materi contained in the peroxide/acidic catalyst decomposition mixture. Again it is disclosed that the decompositio can be promoted by the addition to the residue of acids such as sulphuric acid, phosphoric acid c sulphonic acids, as well as acid-washed activated earth, such as Fuller's earth.

A decomposition catalyst of sulphur dioxide or sulphuric acid is also used in US-A-4016213 to obta phenol and acetone from cumene hydroperoxide.

In US-A-4246203 a hydroperoxide of an aromatic compound is converted into a volatile phenol and carbonyl compound in a cleavage decomposition reaction. A wide range of both solid and liquid cleavag catalysts may be used, including acetic acid, sulphur dioxide, sulphur, formic acid, phosphoric acid an fluoroboric acid, although sulphuric acid is preferred. Silica/alumina gave rather poor yields of phenol an acetone under these conditions.

Lewis acid catalysts were employed according to US-A-4267380 to decompose cumene hydroperoxid into phenol and acetone. Some Lewis acids were unsatisfactory or, in some instances, found to b catalytically inert. Preferred Lewis acids were tungsten hexafluoride, silicon tetrafluoride stannous chloride stannic fluoride, antimony pentachloride, sulphur monochloride and sulphur tetrafluoride.

US-A-4209465 indicates that cumene hydroperoxide could be decomposed into phenol and aceton using an isolable carbonium, tropylium or oxonium salt, such as triphenylcarbonium tetrafluoroborate, as th catalyst.

According to US-A-4267379, cumene hydroperoxide is decomposed into phenol and acetone usin boron trifluoride, or boron trifluoride complexed with an oxygen-containing polar compound.

US-A-4358618 describes a process for decomposing a cumene oxidation product mixture by mixing with an acid that lowers the cumene hydroperoxide concentration and converts most of the dimethylphen carbinol into dicumyl peroxide.

An article by Augustin et al, in Stud. Univ. Babes- Bolyai , Chem. 1986, 31, 19-23 (see Chem. Abstrac 107: 236170j, 1987), relates to "The Life of Synthetic Alumino-silicate Catalysts In The Decomposition C Cumene Hydroperoxide".

US-A-4743573 describes catalysts for the selective decomposition of cumene hydroperoxide in phenol and acetone which comprises oxide forms of silicon, aluminium and boron in the form of crysta having a zeolite structure wherein aluminium and boron replace silicon in the crystalline structure of silic and wherein the crystals are interconnected by oligomeric silica. The phenol selectivity is typically 80.5 t 96% with these catalysts in batch studies, and more than 98% in continuous synthesis at cumer hydroperoxide conversion levels of 90%.

EP-A-0203632 describes a catalyst for decomposition of cumene hydroperoxide to produce phenol ar acetone, comprising zeolite crystals containing boron and aluminium bonded with silica. A portion of th silicon atoms in the crystal lattices of silica are replaced by Al and B, and the zeolite crystals are bonded each other by a siliceous bonding agent which allows the catalyst to assume the shape of mechanical stable microspheres.

Carboxylic acid derivatives have also been used to catalyze cumene hydroperoxide decomposition, Iz Akad. Nauk Turkm. 5512, Ser. Fiz. - Tekh, Khim, Geol . Nauk 1987, (2), 108-10 (Russ) and Chem. Abstrac

108:55583w (1988).

Molybdenum, vanadium and titanium catalysts have also been used for the catalytic decomposition of cumyl hydroperoxide to yield mainly phenol and acetone, see Stozhkova, G.A., et al . (Yarosl . Plitekh. Inst. Yaroslavl, USSR) Neftekhimiya 1987, 27(1), 137-41 (Russ) and Chem. Abstracts 107:197676g (1987).

The use of clays in catalysis is also known.

The unusual properties of smectite clays which make them of interest as catalysts are discussed in an article titled "Catalysis: Selective Developments", Chem. Systems Report 84-3, 239-249, at Section 3.4320. These compositions are layered and exhibit a 2:1 relationship between tetrahedral and octahedral sites. In addition, the combination of cation exchange, intercalation, and the fact that the distance between layers can be adjusted, provide interesting possibilities.

There is a discussion of clay mineral catalysts, including "acid" montmorillonite clay catalysts in "Progress in Inorganic Chemistry", Vol. 35, p. 41 (1987). The process of "pillaring" this type of catalyst is discussed. Pillaring can convert a clay lamellar solid into a more heat-resistant two-dimensional zeolite material.

GB-A-2179563 discloses the use of modified, layered clay catalysts in reactions capable of catalysis by protons. The particular suitability of montmorillonite clays is discussed.

The use of stabilized pillared interlayered clays as catalysts in reactions capable of catalysis by protons is disclosed in EP-A-0083970.

US-A-4665044 discloses the preparation of modified clays containing 6.1 to 9.8 ferric ions per cell by contacting an aqueous slurry of layered lattice clay with a hydrolyzed solution of ferric ion. The related EP-A-4665045 discloses similar catalysts which contain chromium ions.

In an article in J. Mol. Catal., 27 (1984) 195, Pinnavaia, et al discuss the pillaring and delamination of smectite clay catalysts by polyoxo cations of aluminium. They demonstrate that pore openings of pillared montmorillonite and montronite clays are determined principally by the method used to dry the flocculated reaction products.

In an article titled "Syntheses of Interlamelar Montmorillonitediphenylphosphine Triosmium Cluster Complexes" in Appl. Catal., 1987, 35, 177 Choudary et al . disclose that clays can be used as alternatives to polymers and inorganic oxides as supports for clusters.

EP-A-0250168 discloses a method for production of glycol ethers by reacting an olefin oxide with an alkanol over a cation-exchangeable lamellar clay catalyst, the exchangeable cations of the catalyst being cations of one or more rare earth elements.

Gaaf et al . (J. Chem. Soc. Chem. Comm., 655, 1983) discuss work showing that nickel substituted mica montmorillonite (Ni-SMM) clay can be intercalated successfully with aluminium and silica-alumina oligomers leading to pillared clays; transmission electron microscopy has revealed agglomeration of the particles which leads to suppression of hydroisomerization catalysis.

In J. Am. Chem. Soc., 1985, 107, 4783, Pinnavaia et al . discuss properties of chromia pillared clay catalysts which exhibit gallery heights that are about 3.0 Å larger than those of zirconia and alumina pillared clay catalysts.

There is a good overview of the use of pillared cation-exchanged and acid-heated montmorillonite catalysts in Applied Clay Science, 2 (1987), p. 309.

A catalytic application of smectite clays is discussed in an article titled "Catalysis of Friedel-Crafts Alkylation by a Montmorillonite Doped with Transition-Metal Cations" in Helvetica Chimica Acta, 70 (1987) p. 577. Here a process is disclosed for obtaining catalysts by the exchange of interstitial cations in the K-10 montmorillonite for use in alkylations with halides, alcohols and olefins. It was found that the efficiency of the catalyst bears no apparent relation to the corresponding Lewis acids under homogeneous conditions. Zirconium and titanium gave the best results in this study.

When acidic substances are utilized as the catalysts the yields are satisfactory, but many of these acid catalysts require substantial expenditure for production of phenol and acetone, there are disposal problems with spent acids or their salts, and there are difficulties in achieving >99.9% purity phenol required commercially, because of entrainment or breakthrough of the acids. In addition, by-products such as mesityl oxide, α-methylstyrene, acetophenone and 2-phenyl-2-propanol are produced along with the product and must somehow be removed and processed.

It would be a substantial advance in the art if phenol and acetone could be produced in yields approaching 100% by decomposition over an inexpensive heterogeneous catalyst using mild conditions. A catalyst which worked at high space velocities using mild conditions and yet afforded high selectivities and yields with a smaller percentage of by-products would be particularly advantageous. Furthermore a very active, long life heterogeneous catalyst would also solve the catalyst disposal and acid entrainment problems cited above.

The present invention provides a method for the production of phenol and acetone by the decompo: tion of cumene hydroperoxide, at a temperature of 20 to 150°C and a pressure of 0.1 to 7 MPa, in tl presence of a heterogeneous catalyst selected from:

a heteropoly acid on an inert support,

an ion exchange resin with a sulfonic acid functionality,

an acidic smectite clay,

a fluorine-containing acid on an inert support, and montmorillonite clay modified by a heteropoly acid or t titanium, zirconium or aluminium.

The method of the invention gives essentially quantitative conversion of cumene hydroperoxide und mild conditions with phenol and acetone as the major product fractions. Continuous generation phenol/acetone in a plug flow reactor at LHSV of 1 to 10 have been demonstrated.

The reaction can be represented by the following:

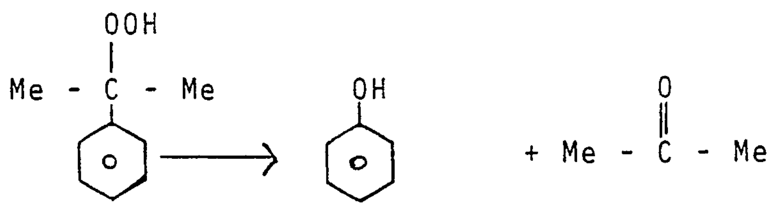

(Eq. 1)

According to one embodiment of this invention the catalyst is a heteropoly acid supported on an ine support or an ion exchange resin having a sulfonic acid functionality. Particularly effective are suc heteropoly acids as tungsto phosphoric, molybdophosphoric, tungstosilicic or molybdosilicic acid, preferab on an inert, high surface area support, such as titania.

A particular advantage of heteropoly acids on titania is that such catalysts have properties which allc for distinct improvements over the use of $H_2SO_4$ and $SO_2$.

The heteropoly acid on titania catalysts are effective in the form of powders, granules or extrudates.

The heteropoly acids comprise a class of acids formed by the condensation of two or more inorgan oxyacids. For example, phosphate and tungstate ions, when reacted in an acidic medium, are condensed form 12-tungstophosphoric acid, a typical heteropoly acid (HPA) according to Equation 2:

$PO_4^{3-} + 12WO_4^{2-} + 27H^+ \rightarrow H_3PW_{12}O_{40} + 12H_2O$ (Eq. 2)

A wide variety of elements ranging from Group I to Group VIII can become the central atom of the HF anion, or the heteroatom as it is called (P in the case of Eq. 2). The nature of the heteroatom is a governir factor which determines both the condensation structure and the physical properties of the HPA.

Atoms coordinated to the heteroatom via oxygen are called polyatoms (W in the case of Eq. 2) and most instances are any one of such limited species as molybdenum, tungsten, niobium and vanadiur When molybdenum is the polyatom, the nature of the heteroatoms, condensation ratios and chemic formulae of the corresponding HPA anions are summarized below.

Anions containing the so-called Keggin structure have a condensation ratio of 1:12 and are the mo typical of all HPA anions. Heterpoly acids with the Keggin structure and their homologues, are generally tl most readily available types of HPA and the ones most commonly used in catalysis. The synthesis of the: HPA's is well documented in the literature (see for example US-A-3947332).

| Typical heteropolymolybdate anions | | |
|---|---|---|
| CONDENSATION RATIOS | HETERO ATOMS(X) | CHEMICAL FORMULAS |
| 1:12 Keggin structure | $P^{5+}$, $As^{5+}$, $Si^{4+}$, $Ge^{4+}$ | $[X^{n+}Mo_{12}O_{40}]^{-(8-n)}$ |
| Silverton structure | $Ce^{4+}$, $Th^{4+}$ | $[X^{4+}Mo_{12}O_{42}]^{8-}$ |
| 1:11 Keggin structure (decomposition) | $P^{5+}$, $As^{5+}$, $Ge^{4+}$, $Si^{4+}$ | $[X^{n+}Mo_{11}O_{39}]^{-(12-n)}$ |
| 2:18 Dawson structure | $P^{5+}$, $As^{5+}$ | $[X_2^{5+}Mo_{18}O_{62}]^{6-}$ |
| 1:9 Waugh structure | $Mn^{4+}$, $Ni^{4+}$ | $[X^{4+}Mo_9O_{32}]^{6-}$ |
| 1:6 Anderson structure (A type) | $Te^{6+}$, $I^{7+}$ | $[X^{n+}Mo_6O_{24}]^{-(12-n)}$ |
| (B type) | $Co^{3+}$, $Al^{3+}$, $Cr^{3+}$ | $[X^{n+}Mo_6O_{24}H_6]$-(6-n) |
| 4:12 | $As^{5+}$ | $[H_4As_4Mo_{12}O_{52}]^{4-}$ |
| 2:5 | $P^{5+}$ | $[P_2Mo_5O_{23}]^{6-}$ |

In the case of conversion of cumene hydroperoxide into phenol/acetone, suitable heterpoly aci catalysts may contain polyatoms selected from the group molybdenum, tungsten, niobium and vanadiurr while the heteroatoms may be phosphorus, silicon, germanium, and arsenic. Preferably the heteroatoms ar phosphorus or silicon. These heteropoly acids have the Keggin structure, $H_{8-n}$ $[XM_{12}O_{40}]$, where X = P c Si , M = Mo or W and n is 4 or 5.

The preferred heteropoly acids include 12-molybdophosphoric acid, $H_3PMo_{12}O_{40}$ , 12-tung stophosphoric acid, molybdosilicic acid, $H_4SiMo_{12}O_{40}$ and 12-tungstosilicic acid. Said acids are generall used as their hydrates; they may be employed by themselves, partially or completely dissolved in th cumene hydroperoxide feed, or they may be employed as heterogeneous catalysts bonded to a suitabl support.

The support should preferably comprise an inert compound. Compounds which may be employed ar those containing elements of Group III and IV of the Periodic Table. Suitable compounds include the oxide of aluminium, silicon, titanium and zirconium or combinations thereof, e.g. alumina, silica (silicon dioxide titania (titanium dioxide) and zirconia, as well as combinations thereof. Also suitable are carbon, ior exchange resins and carbon-containing supports. Good results were observed using $TiO_2$ as the support.

The inert support may be in the form of powders, pellets, spheres, shapes and extrudates. As will b demonstrated by the Examples, the supports are preferably of high purity and high surface area. It ha been found in the process of this invention that greater conversion of cumene hydroperoxide is achieve when the surface area of the support is generally >10 $m^2/g$.

The reaction may be carried out in either a stirred slurry reactor or in a fixed bed continuous flo reactor. The catalyst concentration should be sufficient to provide the desired catalytic effect.

The weight percentage of heteropoly acid relative to Group III/Group IV support should be such that th concentration of the polyatom (Mo, W, Nb or V) in the formulated catalyst is in the range of 0.1 to 30 wt% although concentrations outside this range may also be employed. Where the heteropoly acid is, fc example, 12-molybdophosphoric acid, supported on titania, a suitable quantity of molybdenum is 1 to 1 wt%. In the preparation of a tungstophosphoric acid-on-titania catalyst, on the other hand, the tungste content may be 1 to 30 wt%.

Alternatively, the catalysts comprise a class of ion exchange resins having a strongly acidic catio exchange. These include the gel type or macroreticular ion exchange resin with sulphonic acid ($-SO_3H$), c substituted sulphonic acid functional groups, wherein the sulphonic acid functional group is bonded directl or indirectly to an organic backbone preferably a polystyrene or styrene-divinylbenzene polymer backbone Examples of such resins include AMBERLYST® 15 and XN-1010, AMBERLITE® IR-118, DOWEX® 50 x 2 100 and 5 x 8-100, XL-383 and -386, plus BI0 RAD® AG5OW and AMBERSEP 252H. Another suitable io exchange resin is Rohm & Haas' B-24 high temperature resin, as well as DuPont's NAFION resin, havin perfluoro sulphonic acid functionality. Preferred are the macroporous resins with the styrene divinylbenzen polymer backbone, sulfonic acid functionality, and 1 to 20% cross-linking, such as AMBERLYST 15 an XN-1010. Said resins should be in the acid (hydrogen) form.

According to another embodiment, the catalysts are fluorine-containing compounds on an inert suppor preferably titania. The catalysts are effective in the form of powders, granules or extrudates.

The fluorine impregnated on the catalyst can be present as a hydrogen fluoride group c fluorophosphoric acid group which is chemically bound to the titania support. In the latter instance, th exact nature of the bonding is not fully understood, but is believed to include, for the fluorophosphori

acids-on-titania catalysts, the following:

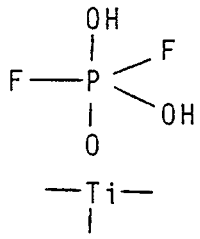 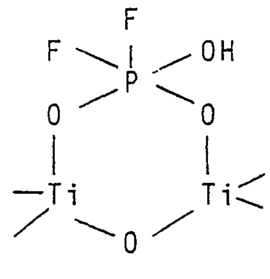 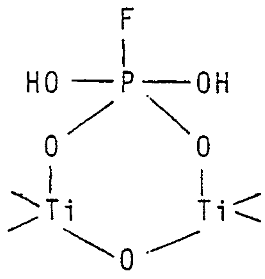

The fluorine may be introduced onto the inert support as a fluorine-containing acid compound. Th same fluorine may, for example, be introduced as a substituted phosphoric acid, such as a fluorophosphori acid, including monofluorophosphoric acid, $FPO_3H_2$, and difluorophosphoric acid, $F_2PO_2H$. Also effectiv are acids such as hydrogen fluoride and aqueous hydrofluoric acid.

The support should preferably comprise an inert compound. Compounds which may be employed ar those containing elements of Group III and IV of the Periodic Table. Suitable compounds include the oxide of aluminium, silicon, titanium and zirconium or combinations thereof, e.g. alumina, silica (silicon dioxide titania (titanium dioxide) and zirconia, as well as combinations thereof. Also suitable are carbon, ior exchange resins and carbon-containing supports. Good results were observed using $TiO_2$ as the support.

The inert support may be in the form of powders, pellets, spheres, shapes and extrudates. As will b demonstrated by the examples, the supports are preferably of high purity and high surface area. It has bee found in the process of this invention that greater conversion of cumene hydroperoxide is achieved whe the surface area of the support is generally $>10\ m^2/g$.

The weight percentage of fluorine to Group III/Group IV support should be such that the concentratio of the fluorine-containing compound in the formulated catalyst is in the range of 0.1 to 20 wt%, althoug concentrations outside this range may also be employed. When the fluorine is, for example, provided b difluorophosphoric acid, supported on titania, a suitable quantity of fluorine is 0.1 to 10 wt%.

In another embodiment, the catalyst comprises an acidic smectite clay, preferably a montmorillonit silica-alumina clay in powdered, granular or extruded form.

A variety of clay catalysts containing aluminium and silica are effective, but it is necessary that the cla be acidic under normal operating conditions. Chemically clays are composed primarily of silicon, aluminiun and oxygen with minor amounts of magnesium and iron in some instances. Variations in the ratios of thes constituents, and their crystal lattice configurations, result in some fifty separate clays, each with its ow characteristic properties.

Smectite clays are discussed in the above-mentioned article cited in Chem. Systems Report, 84-3 These clays have small particle size and unusual intercalation properties which afford them high surfac area. They are aluminosilicates with a unique structure that permits modifications which provide usefu catalysts. They comprise layered sheets of octahedral sites between sheets of tetrahedral sites, and th distance between the layers can be adjusted by swelling, through treatment with the appropriate solvent, c treatment with a pillaring or Lewis acid reagent etc. What renders the smectites of particular interest amon the clay minerals is their combination of cation exchange, intercalation and swelling properties.

The three-layer sheet types of smectite clays include montmorillonite, vermiculite and certain micas, a of which may be expanded between their layers by the appropriate treatment. The idealized basic structur of clays of this type is that of a pyrophyllite which has the basic formula $Si_8Al_4O_{20}(OH)_4$.

A general representation of the montmorillonite structure is:

$M_{x/n}^{n+}\ \text{-}yH_2O(Al_{4-x}Mg_x)(Si_8)O_{20}(OH)_4$

where M represents the interlamellar (balancing cation), normally sodium or lithium and x, y and n ar numbers.

These montmorillonite clays are best used in the present application in an acidic form. Acids activat montmorillonites by attacking and solubilizing structural cations in the octahedral layers. This opens up th clay structure and increases surface area. These acid treated clays act as strong Bronsted acids.

Acidic montmorillonite clays are the preferred form of smectite clay in the present invention. Preferabl these acid clays should have acidities in the range of 3 to 15 mg KOH/gm (or even higher) titrated to phenolphthalein end point. Their surface area should be $>30\ m^2/g$, and preferably 200 to 1000 $m^2/g$. The moisture content should be limited also, whereby upon heating to 220° F (104° C) the weight loss i generally less than 20 wt%.

Illustrative examples of suitable montmorillonite clays include powdered clays such as Filtrol Grade 1: 113 and 160, sold by Harshaw-Filtrol, clays in granular form, such as Filtrol grade 24, having a 20-60 mes size (250 to 841 µm) and grade 25 (10/20 mesh) (841 µm to 2 mm) sold by Harshaw-Filtrol , as well a extruded clays such as the Filtrol Clay-62, sold in 1/16" (1.6 mm) and 3/16" (4.8 mm) diameter extrudates.

In a further embodiment, the catalyst comprises a montmorillonite silica-alumina clay modified by us of a heteropoly acid or by titanium, zirconium or aluminium.

Any of the above-mentioned heteropoly acids may be used in this embodiment of the invention, th preferred heteropoly acids including 12-molybdophosphoric acid, $H_3PMo_{12}O_{40}$, 12-tungstophosphori acid, molybdosilicic acid, $H_4SiMo_{12}O_{40}$ and 12-tungstosilicic acid. Said acids are generally used as the hydratges and the clay is added to the aqueous solution in granular form. Stirring is maintained for 1 to days at room temperature. The mixture is then filtered, the solids washed with distilled water until th washings contain no detectable levels of heteropoly acid and the final product is dried in vacuo at 40° C.

Furthermore, it has been surprisingly discovered that the generation of phenol/acetone by commerciall available acidic montmorillonite clays such as those outlined above is improved significantly by modificatio of the clays with a Group III or IV compound, preferably zirconium, titanium or aluminium. The rate c reaction exhibits between a 6-fold and 10-fold improvement.

The preparation of the zirconium, titanium or aluminium-modified clay catalyst is accomplished b treating an acidic montmorillonite clay, for example Engelhard Clay-24, with an aqueous or alcoholi solution of a salt of the Group III or IV metal and an inorganic acid. For example, granular montmorillonit clay can be added to an aqueous or alcoholic solution or suspension or zirconium(IV) chloride, titanium(I\ chloride or aluminium nitrate. Said salts may be partially hydrolyzed during this addition. Stirring is typicall maintained for 1 to 2 days at about room temperature, but this period can be shorter. The mixture is the filtered, the solids washed until the washings no longer show detectable levels of metal ions, and the fin product dried in vacuo at 40° C.

A further embodiment of this invention involves a novel method for the regeneration of sper montmorillonite clay catalysts. It has been discovered that these catalysts can be regenerated by:

1) Treatment with concentrated nitric acid, or by treatment with methanol.

2) Treatment in this manner is surprisingly effective in that, after treatment with nitric acid, i particular, the catalyst demonstrates:

a) Improved phenol productivity;

b) Improved levels of cumene hydroperoxide conversions;

c) The catalyst exhibits higher ash content and therefore lower levels of organic contaminants;

d) The catalyst exhibits an improved colour which indicates lower levels of organic polymers;

e) In some instances the regenerated clay catalyst has a higher activity than the control samples c fresh catalyst.

Cumene hydroperoxide decomposition may be conducted batchwise, in a continuous slurry bed reacto or in a fixed-bed, continuous flow, reactor. For practical reasons a fixed-bed process is preferred. In a instances, the catalyst concentration should be sufficient to provide the desired catalytic effect.

Cogeneration of phenol and acetone can generally be conducted at temperatures from 20 to 150° C; th preferred range is 40 to 120° C. The operating pressure may be from zero to 1000 psig (0.1 to 7 MPa) c higher. The preferred pressure range is 100 to 400 psig (0.8 to 2.86 MPa) . Because of the highl exothermic nature (52 kal/mole) of the cumene hydroperoxide decomposition (Eq. 1), temperature control i particularly important, especially in a fixed catalyst process.

Yields and purity of product vary according to the catalyst, the reaction conditions and the purity of th starting material, but typically, phenol is generated continuously in up to 50 wt%, often up to ca. 60 wt% concentration in the crude product liquid effluent, and likewise, acetone may be generated in 30 to 50 wt% e.g. 40 wt%, concentrations. The cumene hydroperoxide should preferably be as pure as possible, but a 6 to 80% purity is certainly acceptable. Typical impurities in such an "80%" cumene hydroperoxide feed ar cumene, 2-phenyl-2-propanol and acetophenone. The cumene hydroperoxide is generally diluted with a inert solvent, or with product, before being fed to the decomposer. Typical diluents include acetone, or mixture of acetone, cumene and/or phenol

Generally cumene hydroperoxide conversions are quantitative in continuous unit operations. Phen yields, based on hydroperoxide charged, are >99 mole %. Likewise, acetone yields are also 99 mole % c higher.

These yields are acheived at total liquid hourly space velocities (LHSV) of 1 to 10 under mil conditions. Continuous generation of phenol/acetone in a plug flow reactor at values of LHSV from 1 to 1 have been demonstrated, with inter alia AMBERLYST XN-1010 resin, with 12-molybdophosphoric acid-or titania, and with fluorophosphoric acid on titania.

LHSV is defined as follows:

$$LHSV - \frac{\text{Weight Of Total Liquid Feed Run Through The Reactor Per Hour}}{\text{Volume Of Catalyst In Reactor}}$$

Conversion of cumene hydroperoxide (wt%) is estimated in the following Examples using the equation:

$$\frac{\text{Wt\% Conc. of } (C_6H_5C(CH_3)_2OOH \text{ in Feed - Wt\% Conc. of Cumene Hydroperoxide in product}}{\text{Wt\% Conc. of Cumene Hydroperoxide in Feed}} \times 100$$

Yields of phenol/acetone ($C_6H_5OH/CH_3COCH_3$, mole %) are estimated from:

$$\frac{\text{Moles of Phenol or Acetone in Product Liquid}}{\text{Moles of Cumene Hydroperoxide in Feed}} \times 100$$

The Examples which follow illustrate the cogeneration of phenol and acetone from cumene hydroperox ide using heterogenous catalysts, and the production of such catalysts.

## EXAMPLE A

This example illustrates the preparation of a typical heteropoly acid-on-titania catalyst.

To a solution of 12-tungstophosphoric acid (40.0g) in water (150 ml ) was added, with stirring, 125 ml c HSA titania carrier extrudate (from Norton Company, 64467, 1/8" (3.2 mm) extrudates, surface area ca. 6( $m^2/g$) . The liquid was absorbed into the extrudates, with stirring, for 1 to 2 hours. The mixture was the rotary evaporated to remove excess liquid and calcined at 150 to 350 °C in a stream of nitrogen.

Weight of recovered, white, extrudates: 150.8g

Analysis: W = 17.0%

P = 0.6%

## EXAMPLE B

This example illustrates the preparation of another typical heteropoly acid catalyst.

To a solution of 12-molybdophosphoric acid (30.0g) in water (250 ml) was added, with stirring, 375 cc c HSA titania carrier extrudate (from Norton Company, 64775, 51 $m^2/g$ surface area). The liquid was absorbe into the extrudates, with stirring for 1 to 2 hours. The mixture was then rotary evaporated to remove exces liquid and calcined at 150 to 350 °C in a stream of nitrogen.

Weight of recovered, yellow, extrudates: 252g

Analysis: Mo = 2.8%

P = 0.09%

## EXAMPLE 1

The data in Example 1 illustrate the quantitative conversion of cumene hydroperoxide int phenol/acetone catalyzed by the tungstophosphoric acid on titania catalyst of Example A. Reaction i conducted under mild conditions. Minor by-products include alpha-methylstyrene; cumene, acetophenon and 2-phenylpropanol, and are present in the cumene hydroperoxide feed and apparently remain to som extent in the mixture during peroxide decomposition.

A 250 ml round bottom flask fitted with a condenser, heater, stirrer and feed control , was charged wit

a mixture of 60.g of acetone and 5.0g of 12-tungstophosphoric acid on titania (5972-83R, 17%W). Th mixture was heated to reflux (57° C) with stirring, and 40.0g of 80% cumene hydroperoxide solution fee (having the composition given below) were added dropwise at a rate such that the pot temperature did nc exceed 80° C. After the peroxide addition was complete, the mixture was heated under reflux for a additional 2 hours.

Upon cooling, the product mix was weighed (104.1g), and the product liquid (volume 113 ml ) wa neutralized with sodium bicarbonate and analyzed by GLC.

The product had the following composition (wt%) :

| | |
|---|---|
| Acetone | 70.9 |
| Phenol | 19.6 |
| Cumene | 6.7 |
| Alpha-methyl styrene | 1.4 |
| 2-phenyl-2-propanol | 0.3 |
| Acetophenone | <0.1 |
| Cumene hydroperoxide | <0.1 |

| Feed Composition: | |
|---|---|
| Cumene hydroperoxide | 78.5 |
| Cumene | 16.5 |
| 2-phenyl-2-propanol | 4.7 |
| Acetophenone | 0.4 |

Estimated yield of phenol (based on cumyl hydroperoxide charged) is >99 mole %.

## EXAMPLES 2 to 14

Following the procedures of Example 1, the cogeneration of phenol plus acetone has been dem onstrated using a series of heteropoly acid catalysts and sulphonic acid resins. The results are summarize in Table I.

It may be noted that phenol/acetone generation from the same 80% cumene hydroperoxide as Exampl 1 has been demonstrated using as catalysts:

a) A variety of heterogeneous heteropoly acids, including tungstophosphoric acid, molyt dophosphoric acid, tungstosilicic acid and molybdosilicic acid, each supported on titania (see Examples 1 t 6).

b) A variety of different resins having sulphonic acid functionality, including Rohm & Haas AMBERLYST XN-1010, AMBERLITE IR-118, AMBERLYST 15, NAFION resin and a high temperature resi B-24 from Rohm & Haas (see Examples 7 to 11).

It may also be noted from a review of the data in Table 1 that:

1) Resins such as DUOLITE C-467 having phosphoric acid functionality are not effective for th desired cumene hydroperoxide decomposition reaction to phenol/acetone (see Example 12) under thes screening conditions.

2) Other acidic catalysts such as phosphoric acid-on-titania and boric acid, prepared by methoc similar to that of Example A, are far less effective for cumene hydroperoxide conversion to phenol/acetor (Examples 13 and 14).

3) Both the heteropoly acid and resin-type catalysts convert much of the 2-phenyl-2-propanol in th cumene hydroperoxide feed (see Example 1) into phenol/acetone

## TABLE 1

| Ex. 1 | Catalyst | Product Composition (Wt%) Acetone | Mesityl Oxide | Cumene | α-Methyl Styrene | Phenol | 2-Phenyl-2-Propanol | Aceto-phenone | Cumene Hydro-peroxide |
|---|---|---|---|---|---|---|---|---|---|
| 1 | W-P/TiO2[a] | 70.9 | 0.3 | 6.7 | 1.4 | 19.6 | 0.3 | - | - |
| 2 | Mo-P/TiO2[b] | 70.6 | - | 6.9 | 1.8 | 20.0 | 0.3 | - | - |
| 3 | 1/2(Mo-P/TiO2) | 69.4 | - | 7.2 | 2.1 | 20.7 | 0.1 | 0.3 | - |
| 4 | 1/4(Mo-P/TiO2) | 68.5 | - | 7.6 | 2.0 | 21.3 | 0.2 | 0.3 | - |
| 5 | Mo-Si/TiO2[c] | 71.5 | - | 6.8 | - | 19.5 | 0.3 | - | - |
| 6 | W-Si-TiO2[d] | 69.0 | 0.1 | 7.3 | 1.7 | 21.0 | 0.1 | 0.3 | - |
| 7 | AMBERLYST® XN-1010[e] | 70.5 | 1.5 | 6.7 | 2.1 | 18.3 | 0.2 | 0.3 | - |
| 8 | AMBERLITE® IR-118[f] | 69.7 | 0.2 | 7.3 | 1.9 | 19.7 | 0.5 | 0.3 | 0.1 |
| 9 | AMBERLYST® 15 | 67.8 | 1.2 | 7.4 | 2.3 | 20.5 | 0.2 | 0.3 | - |
| 10 | NAFION® Resin | 67.2 | 2.8 | 7.1 | 1.5 | 20.3 | 0.1 | 0.3 | - |
| 11 | Resin B-24[g] | 69.8 | - | 6.9 | 2.3 | 19.4 | 0.4 | 0.4 | - |
| 12 | Duolite C-467[h] | 589 | - | 6.7 | - | - | 2.2 | 0.2 | 31.9 |
| 13 | H3PO4/TiO2 | 63.8 | - | 7.0 | - | 3.6 | 2.2 | 0.2 | 23.2 |
| 14 | H3BO3/TiO2 | 57.9 | - | 6.8 | - | - | 2.4 | 0.2 | 32.7 |

[a]Tungstophosphoric acid on titania, 17.0% W
[b]Molybdophosphoric acid on titania, 2.2% Mo
[c]Molybdosilicic acid on titania, 10.0% Mo
[d]Tungstosilicic acid on titania
[e]AMBERLYST® XN-1010 from Rohm & Haas
[f]AMBERLITE® IR-118 from Rohm & Haas
[g]Experimental high temperature resin from Rohm & Haas
[h]Duolite C-467 from Rohm & Haas

## EXAMPLES 15 to 19

To a 150 ml capacity, continuous, plug-flow reactor, fitted with heating, cooling, and feed control, wa charged Rohm & Haas AMBERLYST XN-1010 resin. The resin was pretreated with acetone at 60 °C an then fed with a mixture of acetone (2100g) and 80% cumene hydroperoxide (900g) at various flow rate (150 to 600 g/hr, LHSV: 1 to 4), reactor bed temperatures (60 to 80 °C) and pressures (2.17 to 4.24 MPa) The results are summarized in Table II.

The data show almost quantitative conversion of cumyl hydroperoxide over the full range of condition evaluated in this work. Due to the exothermic nature of hydroperoxide decomposition, the temperature c the reactor bed varied considerably over its length; the observed range is given in col . 3 of Table II.

From these data it may be noted that:

a) The estimated yield of phenol , based on cumyl hydroperoxide charged, is: 98 mole %.

b) A substantial portion of the 2-phenyl-2-propanol in the feed is also converted into product.

c) There is a significant quantity of mesityl oxide by-product formed with this catalyst.

d) Cumene hydroperoxide conversion is essentially quantitative at the lower operating temperatur (60 °C).

TABLE II

| Ex. | Oper Temp (°C) | Bed Temp (°C)[a] | Press (MPa) | Rate (g/hr) | Sample | Product Composition | | | | | | | |
|---|---|---|---|---|---|---|---|---|---|---|---|---|---|
| | | | | | | Acetone | Mesityl Oxide | Cumene | Phenol | α-methyl Styrene | Aceto-Phenone | 2-Phenyl 2-Propanol | Cumene Hydroperoxide |
| 15 | 60 | 57-80 | 2.17 | 150 | 3 | 72.4 | 4.1 | 5.4 | 15.0 | 1.5 | 0.3 | 0.2 | - |
| | | 56-74 | - | - | 5 | 74.9 | 3.4 | 5.0 | 14.1 | 1.4 | 0.3 | 0.1 | - |
| 16 | 60 | 55-84 | 2.17 | 300 | 1 | 69.5 | 3.6 | 6.2 | 17.7 | 1.8 | 0.4 | 0.2 | - |
| | | 56-79 | - | - | 5 | 69.1 | 3.4 | 6.3 | 18.0 | 1.8 | 0.4 | 0.2 | - |
| 17 | 60 | 56-78 | 2.17 | 600 | 9 | 69.6 | 2.3 | 6.3 | 16.8 | 1.7 | 0.4 | 0.3 | 2.3 |
| | | 54-75 | - | - | 13 | 69.8 | 1.9 | 6.2 | 15.5 | 1.5 | 0.3 | 0.4 | 3.9 |
| 18 | 80 | 80-105 | 2.17 | 150 | 15 | 62.9 | 7.1 | 6.7 | 18.0 | 2.5 | 0.9 | 0.2 | 0.2 |
| | | 80-100 | - | - | 18 | 72.3 | 5.2 | 5.1 | 13.5 | 1.8 | 0.6 | 0.2 | 0.3 |
| 19 | 80 | 80-92 | 4.24 | 150 | 21 | 72.7 | 4.8 | 5.1 | 13.1 | 1.7 | 0.5 | 0.2 | 0.8 |
| | | 80-90 | - | - | 24 | 71.7 | 4.6 | 54 | 13.5 | 1.8 | 0.5 | 0.2 | 1.4 |

[a]Catalyst : AMBERLYST® XN-1010

EXAMPLE 20

Following the procedures of Example 1, a sample (40.0g) of 80% cumene hydroperoxide solution acetone (60.0g) was converted into phenol/acetone in the presence of 12-tungstosilicic acid-on-sili catalyst, prepared according to the procedure of Example A using 12-tungstosilicic acid and silica (unit catalysts, 4 mm x 4 mm) and having a 16.0 wt% tungsten loading.

An analysis of the crude product solution showed the presence of (wt%):

| | |
|---|---|
| Acetone | 64.2 |
| Phenol | 22.6 |
| Cumene | 8.3 |
| α-methylstyrene | 3.1 |
| 2-phenyl-2-propanol | 0.1 |
| Acetophenone | 0.3 |
| Cumene Hydroperoxide | <0.1 |

Estimated yield of phenol (based on cumyl hydroperoxide charged) is 99 mole %.

EXAMPLE 21

This example illustrates the generation of phenol/acetone in continuous unit equipment using tl sulphonic acid resin catalyst, AMBERLYST XN-101 and a cumene hydroperoxide feed stream diluted wi acetone/cumene/phenol mixture.

Employing the reactor and procedures of Example 15, 150 cc of AMBERLYST XN-1010 was pretreate with acetone (150 cc/hr) at 60 °C, then fed with a mixture of "80%" cumene hydroperoxide (900 composition as in Example 1) diluted with acetone/cumene/phenol (6.4:1.4:9.2 weight ratio, 2100g) at a fee rate of 150 cc/hr. Operating temperature and pressure were 60 °C and 2.17 MPa. Samples of crude liqu product effluent were collected and analyzed. Results are summarized in Table III.

It may be noted that in typical samples, e.g. Sample 18:

Estimated phenol yield = >99 mole %

Estimated acetone yield 88 mole %

Cumene hydroperoxide conversion was essentially complete.

TABLE III

| Ex. | Catalyst | Temp (°C) | (g/hr) | Sample | Crude Product Composition (wt%) | | | | | | | | |
|---|---|---|---|---|---|---|---|---|---|---|---|---|---|
| | | | | | Acetone | Mesityl Oxide | Cumene | Phenol | Methyl Styrene | Aceto-Phenone | 2-Phenyl 2-Propanol | Cumene Hydroperoxide | 4-Cumyl Phenol |
| 21 | XN-1010[a] | 60 | 150 | 1 | 30.7 | 2.3 | 13.4 | 51.1 | - | 0.2 | - | - | 1.7 |
| | | | | 3 | 28.0 | 2.4 | 13.8 | 53.5 | - | 0.2 | - | - | 1.5 |
| | | | | 4 | 30.7 | 2.2 | 13.3 | 51.6 | - | 0.2 | - | - | 1.6 |
| | | | | 5 | 27.6 | 2.3 | 13.8 | 53.7 | 0.1 | 0.2 | 0.1 | - | 1.9 |
| | | | | 9 | 31.1 | 1.8 | 13.4 | 51.1 | 0.2 | 0.2 | - | - | 1.9 |
| | | | | 10 | 31.4 | 1.8 | 13.4 | 51.0 | 0.2 | 0.2 | - | - | 1.6 |
| | | | | 11 | 31.7 | 1.6 | 13.9 | 50.8 | 0.2 | 0.2 | - | - | 1.2 |
| | | | | 12 | 31.4 | 1.6 | 13.9 | 50.9 | 50.9 | 0.2 | - | 0.2 | 1.4 |
| | | | | 13 | 24.7 | 1.7 | 14.7 | 56.5 | 0.3 | - | 0.2 | - | 1.3 |
| | | | | 14 | 24.9 | 1.5 | 14.7 | 56.5 | 0.3 | 0.2 | 0.1 | - | 1.5 |
| | | | | 18 | 31.6 | 1.2 | 13.9 | 51.2 | 0.4 | - | 0.2 | - | 1.1 |
| | | | | 20 | 31.9 | 1.1 | 13.9 | 51.1 | 0.4 | - | 0.2 | - | 1.1 |
| FEED: | | | | | 24.1 | | 13.3 | 36.7 | | 0.1 | 1.5 | 24.3 | |

[a]Rohm & Haas AMBERLYST® XN-1010

## EXAMPLE 22

Following the procedure of Example 21, a fresh batch of AMBERLYST XN-1010 was tested fo phenol/acetone production at higher liquid feed rates (0.4 to 1.5 Kg/h), (LHSV from 3 to 10) at a reacto temperature of 44° C. Results are summarized in Table IV.

It may be noted that with this AMBERLYST XN-1010 catalyst, the conversion of cumene hydroperoxid was incomplete at the highest feed rates and mesityl oxide was a consistent by-product in each of thes runs. Consequently in Sample 18, at a LHSV of 10:
Estimated phenol yield - 55 mole %
Estimated acetone yield = 53 mole %
Cumene hydroperoxide conversion = 56%

TABLE IV

| Ex. | Catalyst | Temp (°C) | Feed Rate (Kg/h) | Sample | Product Composition (wt%) | | | | | | | | |
|---|---|---|---|---|---|---|---|---|---|---|---|---|---|
| | | | | | Acetone | Mesityl Oxide | Cumene | Phenol | Methyl Styrene | Aceto-Phenone | 2-Phenyl 2-Propanol | Cumene Hydroperoxide | 4-Cumyl Phenol |
| 22 | XN-1010[a] | 44 | 0.45 | 1 | 31.2 | 0.9 | 14.9 | 50.5 | 0.2 | 0.2 | - | - | 1.9 |
| | | | | 4 | 32.5 | 0.7 | 14.6 | 50.0 | 0.3 | 0.2 | - | - | 1.5 |
| | | 44 | 0.75 | 8 | 32.9 | 0.6 | 14.5 | 49.9 | 0.4 | 0.2 | 0.1 | - | 1.2 |
| | | | | 10 | 33.0 | 0.4 | 14.6 | 50.0 | 0.4 | 0.2 | 0.1 | - | 1.0 |
| | | | | 12 | 31.7 | 0.4 | 14.7 | 51.2 | 0.4 | 0.2 | 0.2 | - | 0.9 |
| | | 44 | 1.45 | 13 | 31.3 | 0.1 | 15.1 | 47.5 | 0.3 | 0.2 | 0.7 | 4.3 | 0.3 |
| | | | | 17 | 29.6 | 0.1 | 14.9 | 44.0 | 0.3 | 0.1 | 0.8 | 9.8 | 0.3 |
| | | | | 18 | 29.2 | 0.1 | 14.9 | 43.7 | 0.3 | 0.1 | 0.9 | 10.4 | 0.3 |
| FEED: | | | | | 24.5 | | 14.6 | 35.8 | | 0.1 | 1.4 | 23.6 | |

[a]AMBERLYST® 1010 Resin

## EXAMPLE 23

This example illustrates the generation of phenol/acetone in continuous unit equipment using the 1 molybdophosphoric acid-on-titania catalyst of Example B and a cumene hydroperoxide feed stream dilut with acetone/cumene/phenol mixture.

Employing the reactor and procedures of Example 15, 150 ml of 12-molybdophosphoric acid-on-titar were pretreated with acetone (150 ml/h) at 60°C, then fed a mixture of "80%" cumene hydroperoxic (900g, composition as in Example 1) diluted with acetone/cumene/phenol (6.4:2.4:9.1) weight ratio .2100 at a feed rate of 150 ml/h. Operating temperatures and pressures were 60°C and 2.17 MPa. Samples crude liquid product effluent were collected and analyzed. Results are summarized in Table V.

It may be noted that in the 4th day of the run, Sample 14 shows:

Estimated Phenol Yield = 94 mole %

Estimated Acetone Yield = 99 mole %

Cumene hydroperoxide conversion was essentially complete.

TABLE V

| Ex. | Catalyst | Temp (°C) | (g/h) | Sample | Product Composition (wt%) | | | | | | | | | |
|---|---|---|---|---|---|---|---|---|---|---|---|---|---|---|
| | | | | | Acetone | Mesityl Oxide | Cumene | Phenol | Methyl Styrene | Aceto-Phenone | 2-Phenyl 2-Propanol | Cumene Hydroperoxide | 4-Cumyl Phenol | Day |
| 23 | $MoP/TiO_2$[a] | 60 | 150 | 1 | 26.6 | - | 15.0 | 56.4 | 0.3 | - | 0.2 | - | 0.9 | 1 |
| | | | | 2 | 33.3 | - | 14.2 | 50.5 | 0.4 | - | 0.2 | - | 0.9 | |
| | | | | 3 | 33.3 | - | 14.2 | 50.6 | 0.5 | - | 0.2 | - | 0.6 | 2 |
| | | | | 5 | 33.3 | - | 14.2 | 50.5 | 0.6 | 0.2 | - | - | 0.6 | |
| | | | | 7 | 33.3 | - | 14.2 | 50.5 | 0.7 | - | 0.2 | - | 0.5 | 3 |
| | | | | 9 | 33.3 | - | 14.1 | 50.7 | 0.7 | 0.2 | 0.1 | - | 0.5[b] | |
| | | | | 12 | 33.3 | - | 14.0 | 50.7 | 0.8 | 0.2 | 0.1 | - | 0.4 | 4 |
| | | | | 14 | 33.3 | - | 14.2 | 50.6 | 0.8 | 0.2 | 0.1 | - | 0.3 | |
| | | | | 15 | 33.3 | - | 14.1 | 50.6 | 0.9 | 0.2 | 0.1 | - | 0.3[c] | 5 |
| | | | | 16 | 33.4 | - | 14.0 | 50.6 | 0.9 | 0.2 | 0.1 | - | 0.4[d] | |
| FEED: | | | | | 24.4 | | 14.0 | 36.9 | | 0.1 | 1.4 | 23.1 | | |

[a]Molybdophosphoric acid-on-titania, Sample from Example B

TABLE VI

Product Composition (wt%)

EXAMPLE 24

Following the procedures of Example 23, a fresh batch of 12-molybdophosphoric acid-on-titania wa tested for phenol/acetone production at higher liquid feed rates (0.4 to 15 kg/h, LHSV from 3 to 10). Result are summarized in Table VI.

It may be noted that good results were obtained with this catalyst even at an LHSV of 10, and there wa no evidence for methyl oxide by-product formation. Illustrative are the results for Sample 14, where:

Estimated Phenol Yield = >95 mole %
Estimated Acetone Yield = 88 mole %
Cumene Hydroperoxide Conversion = 97%

The data in Example 25 illustrate the quantitative conversion of cumene hydroperoxide int phenol/acetone catalyzed by difluorophosphoric acid-on-titania. The reaction is conducted under mil conditions. Minor by-products include α-methyl styrene. During the run 2-phenyl-2-propanol is als apparently consumed.

Table VII illustrates:

a) The good performance of other fluorine-on-titania catalysts, e.g. $FPO_3H_2/TiO_2$ and $HF/TiO_2$ (Ex. 2 and 27).

b) The superior performance of fluorophosphoric acid-on-titania compared with non-fluoride, phos phoric acid-on-titania (cf. Ex. 25 to 27 vs. Ex. 28).

Table VIII illustrates the continuous generation of phenol/acetone using the fluorophosphoric acid-or titania catalyst and a cumene hydroperoxide feed diluted with acetone. Cumyl hydroperoxide conversion i >75% throughout this run. It is considered significant that very little (no more than 0.1%) mesityl oxide i found in these products, while α-methyl styrene production is only modest, and the concentration of 2 phenyl-2-propanol is lower than in the feedstock.

Table IX illustrates the continuous generation of phenol/acetone using the same fluorophosphoric acic on-titania catalyst and a cumene hydroperoxide feed diluted with phenol/cumene/acetone mixture. Her

cumyl hydroperoxide conversion is essentially quantitative, there is no mesityl oxide formation and on trace quantities of cumyl phenol 2-phenyl-2-propanol in the feed is almost completely consumed.

Table X shows a very similar experiment with the same feedstock, but employing the hydrogen fluorid on-titania catalyst.

Attached also are descriptions of the preparation of the difluorophosphoric acid-on-titani fluorophosphoric acid-on-titania and hydrogen fluoride on-titania catalysts employed in this work (Example 32 to 34).

## EXAMPLE 25

To a 250 ml round bottom flask fitted with a condenser, heater, stirrer and feed control, was charged mixture of 60.0g of acetone and 5.0g of difluorophosphoric acid-on-titania (Example 33, 0.5% F, 0.6% P) The mixture was heated to reflux (57°C) with stirring and 40.0g of "80%" cumene hydroperoxide solutic (see Example 1 for composition) added dropwise at a rate such that the pot temperature did not excee 80°C. After the peroxide addition was complete, the mixture was heated under reflux for 2 hours further.

Upon cooling, the product mix was weighed (103.3g) and the product liquid volume (114 ml ) wa neutralized with sodium bicarbonate and analyzed by glc.

The product had the following composition (wt%) :

| | |
|---|---|
| Acetone | 71.4 |
| Phenol | 19.1 |
| Cumene | 6.9 |
| α-methyl styrene | 0.7 |
| Acetophenone | 0.3 |
| 2-phenol-2-propanol | 1.2 |
| Cumene hydroperoxide | 0.4 |

Estimated cumene hydroperoxide conversion: >98%
Estimated yield of phenol (basis hydroperoxide charged): 97 mole %.

## EXAMPLES 26 to 28

Following tthe procedures of Example 25, three other heterogeneous catalysts were evaluated fc phenol and acetone production from cumyl hydroperoxide. Results are summarized in Table VII.

## EXAMPLE 29

To a 200 ml capacity continuous plug-flow reactor, fitted with heating, cooling and feed control, wa charged a fluorophosphoric acid-on-titania catalyst (Example 32, 1.8% P, 0.5% F, 150 ml ). The cataly was pretreated with acetone at 60°C, and then fed a mixture of acetone (2100g) and 80% cumer hydroperoxide (900g) at 150 g/h, 60°C and 2.17 MPa pressure. The run was continued for 5 days with litt apparent loss in catalyst activity.

The results from glc analysis of samples taken periodically over that 5 day period are summarized Table VIII.

## EXAMPLE 30

To the same 200 ml capacity reactor of Example 29 was charged another sample of fluorophosphor

acid-on-titania catalyst, Example 32 (150 ml). Again the catalyst was pretreated with acetone at 60 °C f about 2 hours, but this time it was followed by a feed mixture of "80%" cumene hydroperoxide (90g) acetone/cumene/phenol (64:24:92, 2100g) . Operating conditions are 60 °C, 2.17 MPa, 150 g/h liquid fee rate. The run was continued for 6 days with one shut down. There was no apparent loss in catalyst activit basis the phenol and acetone estimated to be produced.

The results from glc analysis of samples taken during this 6 day period are summarized in Table IX.

For a typical product sample (No. 4):

Estimated yield of phenol (basis cumene hydroperoxide charged) = 99 mole %.

Estimated yield of acetone (basis cumene hydroperoxide charged) = 95 mole %.

For Product Sample 22:

Estimated Yield of Phenol = >99 mole %

Estimated Yield of Acetone = 99 mole %.

## EXAMPLE 31

The same 200 ml capacity reactor of Example 29 was charged with a sample of hydrogen fluoride-o titania catalyst (Example 34, 150 ml ). Again the catalyst was pretreated with acetone at 60 °C for about hours, followed by a feed mixture of 80% cumene hydroperoxide (900g) in acetone/cumene/phen (64:24:92, 2100g) . Operating conditions were 60 °C, 2.17 MPa, 150 g/h liquid feed rate. The run wa continued for 3 days. Results from glc analysis of samples taken during this period are summarized Table X.

For a typical product sample (No. 8):

Estimated yield of phenol (basis cumene hydroperoxside charged) = 97 mole %.

Estimated yield of acetone (basis cumene hydroperoxide charged) = 93 mole %.

## EXAMPLE 32

### Catalyst preparation

#### Fluorophosphoric Acid-On-Titania

A preformed titanium dioxide catalyst support, Norton 64701, having a surface area of ca. 60 $m^2$/ (178g, 200 ml) was placed in 1 litre round bottom flask. A solution of fluorophosphoric acid (11.5g) in 80 c of reagent acetone (62g) was added. The flask was placed on a rotary evaporator and evacuated aspirator vacuum, then heated at 55 °C for 1 hour with periodic stirring. The resultant mixture was placed a glass tube (2.5 x 7.5 cm) and heated at 150 °C for 30 min. Thereafter, it was heated at 350 °C und nitrogen flow for 2 hours. Both the calcined and uncalcined catalyst contained 2% P by AA.

## EXAMPLE 33

### II. Catalyst preparation

#### Difluorophosphoric Acid-On-Titania

A nearly identical support, Norton 64775 having a surface area of 51 $m^2/g$ (240g, 220 ml) wa dehydrated by heating at 13.3 Pa pressure at 80 °C for 45 min. The support was placed in a polypropyler bowl and a solution of difluorophosphoric acid (17g) in 125 ml of reagent acetone (96g) was poured over The mixture was stirred for 15 minutes with a polypropylene rod, then transferred to a polypropylene beak

and placed in a desiccator where excess solvent was removed under vacuum with periodic removal fro the desiccator for stirring. The mixture was then placed in a 1 litre round bottom flask and heated und vacuum at 80 °C on a rotary evaporator for 1 hour. It was then calcined as above, at 150 °C for 1 hour an under nitrogen flow at 350 °C for 3 hours. The calcined catalyst contained 0.6% phosphorus by AA an 0.5% F by ion chromatography following oxygen combustion.

## EXAMPLE 34

### Catalyst preparation

Hydrofluoric Acid-On-Titania

The same support as in the previous preparation, Norton 64775 (200g) was similarly dried at 110 °C f 1 hour, then treated in the same way as in Example 33 with a solution of 48% hydrofluoric acid (9g) in 8 ml of acetone (66g). The recovered solids were then calcined at 150 °C and 350 °C under a flow nitrogen. Fluorine content is about 1%.

TABLE VII

| Ex. | Catalyst | Product Composition (wt%) | | | | | | | |
|---|---|---|---|---|---|---|---|---|---|
| | | Acetone | Mesityl Oxide | Cumene | α-Methyl Styrene | Phenol | 2-Phenyl 2-Propanol | Aceto phenone | Cumene Hydroperoxide |
| 26 | $FPO_3H_2/TiO_2$[a] | 71.7 | - | 6.8 | 6.8 | 18.7 | 1.1 | 0.3 | 0.5 |
| 27 | $HF/TiO_2$[b] | 72.0 | - | 6.2 | 0.6 | 20.3 | 0.6 | 0.2 | <0.1 |
| 28 | $H_3PO_4/TiO_2$[c] | 63.8 | - | 7.0 | | 3.6 | 2.2 | 0.2 | 23.2 |

(a)Fluorophosphoric acid-on-titania, prepared by the method of Example 32.
(b)Hydrogen fluoride-on-titania, prepared by the method of Example 34.
(c)Phosphoric acid-on-titania.

TABLE VIII

| Ex. | Catalyst | Temp(°C) Dow Therm | Bed | Feed Rate (g/h) | Press (MPa) | Sample | Product Composition (wt%) | | | | | | | | |
|---|---|---|---|---|---|---|---|---|---|---|---|---|---|---|---|
| | | | | | | | Acetone | Mesityl Oxide | Cumene | Phenol | α-Methyl Styrene | Aceto Phenone | 2-Phenyl 2-Propanol | Cumene Hydroperoxide | Day |
| 29 | $FPO_3H_2TiO_2$[a] | 60 | 60-74 | 150 | 2.17 | 2 | 80.6 | - | 3.6 | 13.5 | 1.3 | 0.2 | 0.4 | 0.1 | 1 |
| | | | 60-74 | | | 4 | 80.6 | - | 3.6 | 13.3 | 1.5 | 0.3 | 0.4 | 0.1 | |
| | | | 60-73 | | | 7 | 80.2 | - | 3.7 | 13.5 | 1.4 | 0.3 | 0.5 | 0.2 | 2 |
| | | | 60-73 | | | 11 | 79.4 | 0.1 | 3.8 | 14.0 | 1.4 | 0.3 | 0.6 | 0.2 | |
| | | | 60-72 | | | 15 | 80.4 | - | 3.6 | 13.2 | 1.3 | 0.3 | 0.7 | 0.3 | 3 |
| | | | 60-72 | | | 18 | 80.4 | - | 3.6 | 13.1 | 1.3 | 0.7 | 0.1 | 0.3 | |
| | | 60 | 59-74 | 600 | 2.17 | 1 | 75.1 | 0.1 | 4.2 | 12.9 | 0.5 | 0.3 | 1.3 | 5.4 | 4 |
| | | | 60-75 | | | 4 | | 0.1 | 3.6 | 10.6 | 0.5 | 0.2 | 1.1 | 4.7 | |
| | | | 60-74 | | | 5 | 79.0 | 0.1 | 3.6 | 10.2 | 0.4 | 0.2 | 1.1 | 5.1 | 5 |
| | | | 60-73 | | | 8 | 79.3 | | 3.5 | 10.1 | 0.4 | 0.2 | 1.2 | 5.0 | |
| Feed Composition: | | | | | | | 72.4 | | 3.5 | | | 0.1 | 1.4 | 22.5 | |

[a]Fluorophosphoric acid-on-titania catalyst, prepared by the method of Example 32.

TABLE IX

| Ex. | Catalyst | Temp(°C) Dow Therm | Bed | Feed Rate (g/h) | Sample | Product Composition (wt%) | | | | | | | | | |
|---|---|---|---|---|---|---|---|---|---|---|---|---|---|---|---|
| | | | | | | Acetone | Mesityl Oxide | Cumene | Phenol | α-Methyl Styrene | Aceto-Phenone | 2-Phenyl 2-Propanol | Cumene Hydroperoxide | 4-Cumyl Phenol | Day |
| 30 | $FPO_3H_2TiO_2$[a] | 60 | 60-75 | 150 | 2 | 23.6 | - | 15.3 | 60.6 | | 0.2 | - | - | 0.1 | 1 |
| | | | 60-75 | | 3 | 32.8 | - | 14.3 | 50.9 | 1.4 | 0.2 | 0.1 | - | 0.1 | |
| | | | 60-75 | | 4 | 32.7 | - | 14.2 | 52.6 | | 0.2 | 0.1 | - | - | 2 |
| | | | 60-75 | | 6 | 32.6 | - | 14.2 | 52.7 | | 0.2 | 0.1 | - | - | |
| | | | 60-74 | | 8 | 32.9 | - | 14.1 | 51.0 | 1.5 | 0.2 | 0.1 | - | -[b] | 3 |
| | | | 60-74 | | 10 | 33.1 | - | 14.1 | 50.9 | 1.5 | 0.2 | 0.1 | - | - | |
| | | | 60-73 | | 11 | 32.9 | - | 14.1 | 51.0 | 1.4 | 0.2 | 0.1 | - | - | 4 |
| | | | 60-73 | | 14 | 33.2 | - | 14.1 | 50.8 | 1.5 | 0.2 | 0.1 | - | - | |
| | | | 60-72 | | 15 | 33.2 | - | 14.0 | 50.9 | 1.4 | 0.2 | 0.1 | 0.1 | - | 5 |
| | | | 60-71 | | 18 | 32.7 | - | 14.1 | 51.1 | 1.5 | 0.2 | 0.1 | 0.1 | - | |
| | | | 60-70 | | 22 | 33.0 | - | 14.2 | 50.8 | 1.4 | 0.2 | 0.2 | 0.1 | - | |
| Feed Composition: | | | | | | 23.9 | | 14.0 | 36.9 | | 0.1 | 1.5 | 23.5 | | |

(a) Fluorophosphoric acid-on-titania, prepared by the method of Example 32.
(b) Shut down for weekend.

TABLE X

| Ex. | Catalyst | TemP(°C) Dow Therm | Bed | Feed Rate (g/h) | Sample | Product Composition (wt%) | | | | | | | | | |
|---|---|---|---|---|---|---|---|---|---|---|---|---|---|---|---|
| | | | | | | Acetone | Mesityl Oxide | Cumene | Phenol | α-Methyl Styrene | Aceto-Phenone | 2-Phenyl 2-Propanol | Cumene Hydroperoxide | 4-Cumyl Phenol | Day |
| 31 | $HF/TiO_2$[a] | 60 | 60-73 | 150 | 1 | 32.9 | - | 14.3 | 51.0 | 1.2 | 0.2 | 0.1 | - | 0.2 | 1 |
| | | | 60-72 | | 2 | 31.7 | - | 14.2 | 52.2 | 1.4 | 0.2 | 0.1 | - | - | |
| | | | 60-67 | | 4 | 33.1 | - | 14.0 | 51.0 | 1.4 | 0.2 | 0.1 | - | - | 2 |
| | | | 61-68 | | 7 | 33.2 | - | 14.0 | 51.0 | 1.3 | 0.2 | 0.2 | - | - | |
| | | | 58-63 | | 8 | 32.5 | - | 14.5 | 51.1 | 1.1 | 0.2 | 0.4 | 0.1 | - | 3 |
| | | | 61-66 | | 11 | 32.6 | - | 14.2 | 50.1 | 1.2 | 0.2 | 0.5 | 1.0 | | |
| Feed Composition: | | | | | | 24.3 | | 13.9 | 37.2 | | 0.1 | 1.4 | 23.0 | | |

(a)Hydrogen fluoride-on-titania, prepared by the method of Example 34.

EXAMPLE 35

This Example illustrates the quantitative conversion of cumene hydroperoxide into phenol/aceton catalyzed by Harshaw-Filtrol Clay-24, an acidic montmorillonite clay in granular form. The reaction . conducted under mild conditions. Minor by-products include alpha-methylstyrene, but they are present i much smaller amounts than in the related art; cumene, acetophenone and, 2-phenyl-2-propanol are prese in the cumene hydroperoxide feed and apparently these by-products remain to some extent in the mixtur during peroxide decomposition.

A 250 ml round bottom flask fitted with a condenser, heater, stirrer and feed control was charged with mixture of 60.0g of acetone and 5.0g of Harshaw-Filtrol Clay-24 (acidity 8.5 mg KOH/gm, surface area 42 $m^2/g$), in granular form. The mixture was heated to reflux (57° C) with stirring, and 40.0g of "80%" cumen hydroperoxide solution (see Example 1) were added dropwise at a rate such that the pot temperature di not exceed 80° C. After the peroxide addition was complete, the mixture was heated under reflux for a additional 2 hours.

Upon cooling, the product mix was weighed (104.0g) and the product (volume 110 ml ) was neutralize with sodium bicarbonate and analyzed by glc.

The product had the following composition (wt%) :

| | |
|---|---|
| Acetone | 69.8 |
| Phenol | 19.4 |
| Cumene | 6.9 |
| Alpha-methyl styrene | 2.3 |
| Acetophenone | 0.4 |
| Cumene hydroperoxide | <0.1 |

From these data it may be noted that:

a) The estimated yield of phenol (based on cumyl hydroperoxide charged was: 98 mole %.

b) A substantial portion of the 2-phenyl-2-propanol in the feed was also converted into product.

c) No significant quantity of mesityl oxide by-product was formed with this catalyst.

d) Cumene hydroperoxide conversion was essentially quantitative.

EXAMPLES 36 to 39

Table XI illustrates the generation of phenol/acetone in batch equipment under the same mild conditior as Example 35, but using a variety of other acidic montmorillonite clays. These clays vary in acidity fror 3.0 to 15 mg KOH/gm and are in powder and extruded form.

TABLE XI

| Ex. | Catalyst | Product Composition (wt%) | | | | | | |
|---|---|---|---|---|---|---|---|---|
| | | Acetone | Cumene | α-Methyl Styrene | Phenol | 2-Phenyl-2-Propanol | Aceto-phenone | Cumene Hydroperoxide |
| 36 | Clay-113[b] | 72.3 | 6.8 | 1.8 | 18.7 | 0.1 | 0.2 | - |
| 37 | Clay-13[c] | 70.0 | 7.3 | 1.3 | 20.1 | 1.0 | 0.3 | - |
| 38 | Clay-160[d] | 69.4 | 7.2 | 0.3 | 20.8 | 1.7 | 0.3 | 0.2 |
| 39 | Clay-62[e] | 63.4 | 7.2 | 0.3 | 7.3 | 2.0 | 0.2 | 19.5 |

(a)Run in Batch, Glassware Equipment as in Example 35.
(b)Powder, 10 mg KOH/gm Acidity, Surface Area 300 $m^2/g$, from Harshaw-Filtrol.
(c)Powder, 15 mg KOH/gm Acidity, Surface Area 300 $m^2/g$. from Harshaw-Filtrol
(d)Powder, 13 mg KOH/gm Acidity, Surface Area 330 $m^2/g$, from Harshaw-Filtrol.
(e)Extrudate, 4.75 mm Diameter, 3.0 mg KOH/gm Acidity, Surface Area 275 $m^2/g$, from Harshaw-Filtrol.

## EXAMPLES 40 and 41

These Examples illustrate the generation of phenol/acetone in continuous unit equipment using the Harshaw-Filtrol Clay-24 catalyst.

A 200 ml plug-flow reactor, equipped with Dow-Therm cooling capabilities, and heating, pressure and flow controls was charged with 150 cc of Clay-24 catalyst. The catalyst bed was first pretreated with acetone at 60 °C, then a mixture of "80%" cumene hydroperoxide (CHP of composition shown in Example 1), diluted to 20% concentration with >99% grade acetone, was fed upflow, at two flow rates (150 and 600 ml/h) at a reactor temperature of 60 °C and 2.17 MPa pressure. The crude liquid product was collected and analyzed. Results are summarized in Table XII.

It is considered significant here that in these runs:

a) The cumene hydroperoxide conversion was quantitative over this range of conditions.

b) Acetophenone, 2-phenyl-2-propanol, mesityl oxide and α-methylstyrene concentrations in the crude product were all low.

Due to the exothermic nature of the cumene hydroperoxide decomposition reaction (52 Kcal/mol ), the temperature profile along the catalyst bed in these runs typically ranged from 60 to 100 °C at the beginning of the run, down to 60 - 83 °C under equilibrium conditions.

TABLE XII

| Ex. | Reactor Temp. (°C) | Press (MPa) | Feed Rate (g/h) | Sample | Crude Product Composition (wt%) | | | | | | | |
|---|---|---|---|---|---|---|---|---|---|---|---|---|
| | | | | | Acetone | Mesityl Oxide | Cumene | Phenol | α-Methyl Styrene | Aceto-Phenone | 2-Phenyl 2-Propanol | Cumene Hydroperoxide |
| 40 | 60 | 2.17 | 150 | 2 | 78.7 | 0.3 | 4.3 | 14.3 | 1.4 | 0.5 | 0.1 | - |
| | | | | 6 | 79.1 | 0.1 | 4.3 | 14.3 | 1.5 | 0.4 | 0.1 | - |
| | | | | 8 | 79.5 | 0.2 | 3.7 | 14.7 | 1.4 | 0.3 | - | - |
| | | | | 10 | 79.9 | 0.2 | 3.6 | 14.3 | 1.4 | 0.3 | - | - |
| | | | | 14 | 79.7 | 0.2 | 3.7 | 14.4 | 1.5 | 0.2 | - | - |
| | | | | 18 | 79.6 | 0.1 | 3.7 | 14.7 | 1.4 | 0.2 | - | - |
| 41 | 60 | 2.17 | 600 | 1 | 79.8 | 0.2 | 3.6 | 14.4 | 1.3 | 0.3 | - | - |
| | | | | 5 | 80.4 | 0.1 | 3.6 | 14.0 | 1.4 | 0.2 | - | - |
| | | | | 6 | 80.2 | 0.1 | 3.6 | 14.3 | 1.3 | 0.2 | - | - |
| | | | | 10 | 80.3 | 0.1 | 3.6 | 14.2 | 1.4 | 0.2 | - | - |

[(a)] Run in continuous, Dow Therm jacketed reactor, upflow, with 150 ml of catalyst charge.
[(b)] Catalyst: Harshaw-Filtrol Clay-24, granules (Acidity 8.5 mg KOH/gm, Surface Area 425 $m^2/g$).

## EXAMPLE 42

This Example illustratges the generation of phenol/acetone in continuous unit equipment using the Cla
24 catalyst and a cumene hydroperoxide feedstock diluted with acetone/cumene/phenol mixture.

Employing the reactor and procedures of Example 40, 150 cc of Clay-24 were pretreated with acetor (150 cc/hr) at 60° C, then fed with a mixture of "80%" cumene hydroperoxide (900g, composition as i Example 1) diluted with acetone/cumene/phenol (6.4:2.4:9.2 weight ratio, 2100g) at a feed rate of 150 ml I Operating temperatures and pressures were 60° C and 2.17 MPa. Samples of crude liquid product effluei were collected and analyzed. Results are summarized in Table XIII.

It may be noted that in typical samples, e.g. Sample 8:

Estimated Phenol Yield = >99 mole %

Estimated Acetone Yield = 97 mole %

No significant quantity of mesityl oxide was found in the crude product.

Cumene hydroperoxide conversion is quantitative.

A significant portion of the 2-phenyl-2-propanol in the liquid feed was also converted into products.

Material balance data were consistently 99%, and typically 99.3+%.

Due to the exothermic nature of the peroxide decomposition reaction, the temperature profile along th reactor bed in this run varied from 60 to 93° C at the start to 60 to 73° C under equilibrium conditions.

TABLE XIII

| Ex. | Catalyst | Temp °C | Feed Rate (g/h) | Sample | Crude Product Composition (wt%) | | | | | | | | |
|---|---|---|---|---|---|---|---|---|---|---|---|---|---|
| | | | | | Acetone | Mesityl Oxide | Cumene | Phenol | Methyl Styrene | Aceto-Phenone | 2-Phenyl 2-Propanol | Cumene Hydroperoxide | 4-Cumyl Phenol |
| 42 | Clay-24[a] | 60 | 150 | 2 | 33.1 | - | 14.0 | 50.6 | 0.4 | - | 0.3 | - | 1.0 |
| | | | | 3 | 26.8 | - | 14.8 | 55.8 | 0.4 | 0.3 | - | - | 1.1 |
| | | | | 4 | 25.3 | - | 15.0 | 57.1 | 0.3 | 0.3 | - | - | 1.2 |
| | | | | 7 | 25.9 | - | 15.1 | 56.8 | 0.3 | 0.3 | - | - | 0.9 |
| | | | | 8 | 32.9 | - | 14.0 | 51.2 | 0.3 | 0.2 | - | - | 0.7 |
| | | | | 11 | 32.0 | - | 14.2 | 51.9 | 0.3 | - | 0.2 | - | 0.9 |
| | | | | 12 | 31.1 | - | 14.2 | 52.6 | 0.3 | - | 0.2 | - | 0.9 |
| | | | | 15 | 32.7 | - | 14.0 | 51.5 | 0.3 | - | 0.2 | - | 0.6 |
| | | | | 16 | 33.2 | - | 13.9 | 50.7 | 0.4 | 0.3 | - | - | 0.7 |
| | | | | 17 | 33.2 | - | 14.1 | 50.7 | 0.4 | 0.2 | - | - | 0.8 |
| | | | | 20 | 33.2 | - | 14.1 | 50.7 | 0.4 | 0.2 | - | - | 0.8 |
| | | | | 22 | 33.3 | - | 14.0 | 50.8 | 0.4 | 0.2 | - | - | 0.7 |
| Feed: | | | | | 24.3 | | 13.8 | 36.6 | | 0.1 | 1.4 | 23.2 | 0.1 |

(a)Harshaw-Filtrol Clay-24, Granules.

## EXAMPLE 43

This Example illustrates the generation of phenol/acetone at higher feed rates, up to a LHSV of 10.

Using the reactor and procedures of Example 40 and a feed composition similar to that of Example 4: a fresh sample of Clay-24 catalyst was evaluated at a series of different feed rates (i .e. 0.45, 0.75 and 1. Kg/h, corresponding to liquid hourly space velocities of 3.0, 5.0 and 10.0 respectively).

Results are summarized in Table XIV.

It may be noted that in typical samples, e.g. Sample 17:

Estimated Phenol Yield = 98 mole %

Estimated Acetone Yield = 99 mole %

Cumene hydroperoxide conversion essentially quantitative even at a LHSV of ten.

The temperature profile along the reactor bed typically varied in this run from 60 to 107° C at a LHSV ( 3, to 81 to 105° C at a LHSV of 10.

TABLE XIV

| Ex. | Catalyst | Temp °C | Feed Rate (Kg/h) | Sample | Product Composition (wt%) | | | | | | | | |
|---|---|---|---|---|---|---|---|---|---|---|---|---|---|
| | | | | | Acetone | Mesityl Oxide | Cumene | Phenol | Methyl Styrene | Aceto-Phenone | 2-Phenyl 2-Propanol | Cumene Hydroperoxide | 4-Cumyl Phenol |
| 43 | Clay-24[a] | 60 | 0.45 | 1 | 33.4 | - | 14.2 | 50.4 | 0.9 | - | 0.3 | - | 0.3 |
| | | | | 3 | 33.4 | - | 14.1 | 50.3 | 1.0 | - | 0.3 | - | 0.2 |
| | | | | 6 | 33.4 | - | 14.1 | 50.5 | 0.9 | - | 0.2 | - | 0.3 |
| | | | 0.75 | 7 | 33.5 | - | 14.1 | 50.3 | 1.0 | - | 0.3 | - | 0.2 |
| | | | | 9 | 33.5 | - | 14.1 | 50.4 | 1.1 | - | 0.2 | - | 0.2 |
| | | | | 12 | 33.4 | - | 14.0 | 50.9 | 0.9 | - | 0.2 | - | 0.1 |
| | | | 1.5 | 13 | 33.5 | - | 14.1 | 50.6 | 1.0 | - | 0.3 | 0.1 | 0.3 |
| | | | | 14 | 33.3 | - | 14.1 | 50.7 | 1.0 | - | 0.3 | - | 0.1 |
| | | | | 17 | 33.3 | - | 14.1 | 50.8 | 1.0 | - | 0.2 | - | 0.2 |
| Feed: | | | | | 24.4 | | 14.1 | 37.2 | | 0.1 | 1.5 | 22.7 | |

[a] Harshaw-Filtrol, Clay, Grade-24

EXAMPLE 44

This Example illustrates the longevity or the Clay-24 catalyst when used in phenol/acetone production.

A 50 ml capacity, plug flow, electrically heated reactor equipped with pressure and flow controls, wa charged with 25 ml of Clay-24 catalyst. A liquid mixture of 80% cumene hydroperoxide diluted wit acetone/cumene/phenol (6.4: 2.4:9.2 weight ratio) was pumped through the catalyst bed for a total of 4 days. Operating conditions were 60 °C, 2.17 MPa, 25 ml/h feed rate.

A summary of the results in Table XV shows that catalyst activity remains good over the 43 da (1000 + hr) run period, with no need for catalyst regeneration or replacement.

TABLE XV

| Ex. | Catalyst | Sample | Product Composition (wt%) | | | | | | | | | |
|---|---|---|---|---|---|---|---|---|---|---|---|---|
| | | | Acetone | Mesityl Oxide | Cumene | Phenol | Methyl Styrene | Aceto-Phenone | 2-Phenyl 2-Propanol | Cumene Hydroperoxide | 4-Cumyl Phenol | Time (Days) |
| 44 | Clay-24 | 1 | 33.6 | - | 14.1 | 49.8 | 1.5 | 0.2 | 0.1 | 0.1 | - | 1 |
| | | 2 | 33.4 | - | 14.5 | 50.0 | 0.6 | 0.2 | - | - | 0.8 | 10 |
| | | 3 | 33.5 | - | 14.4 | 49.9 | 0.6 | 0.2 | 0.1 | 0.2 | 0.6 | 22 |
| | | 4 | 33.4 | - | 14.4 | 49.9 | 0.6 | 0.2 | 0.1 | 0.4 | 0.5 | 29 |
| | | 5 | 34.0 | - | 11.9 | 50.2 | 0.8 | 0.4 | 0.4 | 1.4 | 0.5 | 38 |
| | | 6 | 33.3 | - | 13.8 | 50.0 | 0.7 | 0.2 | 0.2 | 1.1 | 0.4 | 43 |
| Feed: | | | 24.8 | | 14.7 | 35.7 | - | 0.1 | 1.4 | 23.2 | | |

## EXAMPLE 45

This Example illustrates the generation of phenol/acetone at a LHSV of up to 60.

The 200 ml capacity, upflow reactor of Example 40 was charged with a 25 ml plug (7.5 to 10 cm lonc of Clay-24 catalyst. After pretreatment with acetone, the reactor was fed a mix of 80% cumene hydroperox ide diluted with acetone/cumene/phenol, as in Example 42, but to a CHP level of only 1.5 wt%. The liqui feed rate was 1.5 Kg/h, the effective LHSV was ca. 60.

Samples of product effluent were analyzed periodically.

Cumene hydroperoxide conversion was essentially quantitative, see Table XVI. Phenol and aceton concentration rose measurably.

TABLE XVI

| Ex. | Catalyst | Temp (°C) | Feed Rate (Kg/h) | Sample | Product Composition | | | |
|---|---|---|---|---|---|---|---|---|
| | | | | | Acetone | Cumene | Phenol | Cumene Hydroperoxide |
| 45 | Clay-24 | 60 | 1.5 | 1<br>3 | 35.2<br>34.9 | 13.9<br>13.9 | 50.7<br>51.0 | -<br>- |
| Feed: | | | | | 34.6 | 13.9 | 49.8 | 1.5 |

## EXAMPLES 46 to 50

These Examples illustrate the syntheses of phenol and acetone using Clay-24 catalyst over a range o temperatures and pressures.

Following the procedure of Example 40, the Clay-24 catalyst was tested using the "80%" cumen hydroperoxide feed in acetone/cumene/phenol over the temperature range of 48 to 80 °C and the operatin pressure range 0.79 to 7 MPa. The results are summarized in Table XVII.

Of note:

a) Cumene hydroperoxide conversion was quantitative over the full range of conditions, even thoug high feed throughputs were employed.

b) At 50 °C operating temperature (Example 46, Sample 3):
Phenol Yield = 97 mole %
Acetone Yield = 99 mole %

c) At 80 °C operating temperature (Example 48, Sample 3):
Phenol Yield = 94 mole %
Acetone Yield = >99 mole %

d) At 0.79 MPa operating pressure (Example 49, Sample 3):
Phenol Yield = 95 mole %
Acetone Yield = 97 mole %

TABLE XVII

| Ex. | Catalyst | Pressure (MPa) | Temp (°C) | Feed Rate (Kg/h) | Sample | Product Composition (wt%) | | | | | | | | |
|---|---|---|---|---|---|---|---|---|---|---|---|---|---|---|
| | | | | | | Acetone | Mesityl Oxide | Cumene | Phenol | Methyl Styrene | Aceto-Phenone | 2-Phenyl 2-Propanol | Cumene Hydroperoxide | 4-Cumene Phenol |
| 46 | Clay-24 | 2.17 | 50 | 1.5 | 1 | 33.8 | - | 13.8 | 49.9 | 0.9 | 0.3 | - | - | 0.8 |
| | | | | | 2 | 31.0 | - | 14.1 | 52.5 | 0.9 | 0.3 | - | - | 0.8 |
| | | | | | 3 | 33.9 | - | 13.7 | 50.2 | 0.8 | 0.3 | - | - | 0.7 |
| 47 | Clay-24 | 2.17 | 48 | 1.5 | 4 | 34.3 | - | 13.7 | 49.6 | 0.8 | 0.2 | - | - | 0.6 |
| | | | | | 5 | 31.3 | - | 14.0 | 52.2 | 0.9 | 0.3 | - | - | 0.6 |
| | | | | | 6 | 34.1 | - | 13.6 | 50.0 | 0.8 | 0.2 | - | - | 0.6 |
| 48 | Clay-24 | 2.17 | 80 | 1.5 | 1 | 34.1 | - | 13.8 | 49.6 | 1.0 | 0.3 | - | - | 0.7 |
| | | | | | 2 | 31.3 | - | 14.2 | 51.8 | 1.0 | 0.3 | - | - | 0.8 |
| | | | | | 3 | 34.2 | - | 13.8 | 49.5 | 0.9 | 0.3 | - | - | 0.7 |
| 49 | Clay-24 | 0.79 | 60 | 1.5 | 1 | 34.0 | - | 13.8 | 49.8 | 0.9 | 0.3 | - | - | 0.6 |
| | | | | | 2 | 30.3 | - | 14.3 | 52.8 | 0.9 | 0.3 | - | - | 0.6 |
| | | | | | 3 | 33.8 | - | 13.8 | 49.9 | 0.9 | 0.3 | - | - | 0.6 |
| 50 | Clay-24 | 7 | 60 | 1.5 | 4 | 34.1 | - | 13.7 | 49.7 | 0.8 | 0.3 | - | - | 0.6 |
| | | | | | 5 | 29.9 | - | 14.3 | 53.2 | 0.9 | 0.3 | - | - | 0.6 |
| | | | | | 6 | 34.1 | - | 13.7 | 49.8 | 0.9 | 0.3 | - | - | 0.6 |
| | Feed: | | | | | 24.6 | - | 13.7 | 35.7 | - | 0.1 | 1.6 | 23.9 | - |
| | | | | | | 24.7 | - | 13.8 | 35.5 | - | 0.2 | 1.5 | 24.0 | - |
| | | | | | | 24.6 | - | 13.6 | 35.2 | - | 0.1 | 1.6 | 24.5 | - |
| | | | | | | 24.9 | - | 13.7 | 35.7 | - | 0.1 | 1.6 | 24.0 | - |

EXAMPLE 51

This Example illustrates the use of an extruded clay catalyst in continuous acetone/phenol service.

Following the procedures of Example 40, the reactor was charged with 150 cc of Harshaw-Filtrol Cla 62, 1.6 mm diameter extrudates. The typical feed, "80%" cumene hydroperoxide diluted w acetone/cumene/phenol, was pumped through the bed at a reactor temperature of 80 °C and flow rates between 3 and 10 LHSV. Analyses of effluent samples are summarized in Table XVIII.

It may be noted that:

a) Cumene hydroperoxides were typically 96 to 100% per pass.

b) For Sample 7:

Phenol Yield = 94 mole %

Acetone Yield = >99 mole %

At the LHSV of 3, the temperature profile along the reactor bed was typically 80 to 108 °C; at LHSV 10, the typical range was up to 118 °C.

TABLE XVIII

| Ex. | Catalyst | Pressure (MPa) | Temp (°C) | Feed Rate (Kg/h) | Sample | Product Composition (wt%) | | | | | | | | |
|---|---|---|---|---|---|---|---|---|---|---|---|---|---|---|
| | | | | | | Acetone | Mesityl Oxide | Cumene | Phenol | Methyl Styrene | Aceto-Phenone | 2-Phenyl 2-Propanol | HydroPeroxide | Cumene 4-Cumyl Phenol |
| 57 | Clay-62 | 2.17 | 80 | 0.45 | 1 | 28.6 | - | 14.2 | 54.7 | 1.0 | 0.3 | 0.1 | - | 0.6 |
| | | | | | 2 | 34.5 | - | 13.6 | 49.5 | 0.9 | 0.2 | 0.1 | - | 0.6 |
| | | | | | 3 | 34.0 | - | 13.5 | 50.1 | 0.9 | 0.2 | 0.1 | - | 0.6 |
| | | | | 0.75 | 4 | 34.3 | - | 13.5 | 49.9 | 0.9 | 0.2 | 0.1 | 0.1 | 0.5 |
| | | | | | 5 | 27.5 | - | 14.2 | 55.9 | 1.0 | 0.3 | 0.1 | 0.1 | 0.6 |
| | | | | | 6 | 34.3 | - | 13.6 | 49.9 | 0.9 | 0.2 | 0.1 | 0.1 | 0.5 |
| | | | | 1.5 | 7 | 33.8 | - | 13.4 | 50.3 | 0.9 | 0.2 | 0.1 | 0.4 | 0.4 |
| | | | | | 8 | 32.2 | - | 13.7 | 51.6 | 0.8 | 0.2 | 0.1 | 0.6 | 0.4 |
| | | | | | 9 | 33.9 | - | 13.5 | 50.0 | 0.8 | 0.2 | 0.1 | 0.8 | 0.4 |
| | Feed: | | | | 3 | 25.4 | - | 13.5 | 38.1 | - | 0.1 | 1.6 | 21.1 | - |

The data in Examples 52 to 58 illustrate:

a) The preparation of 12-tungstophosphoric acid-modified clay (Example 52).

b) Phenol/acetone generation using an unmodified montmorillonite clay catalyst in a typical batc synthesis with a small (standard) quantity of catalyst charged (see Comparative Example AA).

· c) A second Comparative Example demonstrates a lack of reaction in the absence of any cla catalyst (Comparative Example BB)

d) Phenol/acetone generation using the 12-tungstophosphoric acid-modified Clay-24 catalyst ( Example 52. This gives at least a 6-fold improvement in rate of comparison with the unmodified cla (compare Example 53 and Comparative Example AA).

e) Phenol /acetone generation using a series of three other heteropoly acid modified Clay-2 catalysts, prepared by the procedure of Example 52 and tested by the procedure of Example 53. Thes catalysts, including 12-molybdophosphoric acid, 12-molybdosilicic acid and 12-tungstosilicic acid on Cla 24, typically give a 6-fold improvement in rate of comparison with the unmodified clay (compare Example 54 to 56 with Comparative Example AA).

f) Preparation of a 12-tungstophosphoric acid modified Clay-62, in extruded form (Example 57).

g) The use of the 12-tungstophosphoric acid clay extrudates in the continuous generation ( phenol/acetone at high space velocities and low operating temperatures (see Example 58). Here phenol an acetone yields are ≥99 mole %.

## EXAMPLE 52

### Preparation of 12-Tungstophosphoric Acid Modified Clay

To a 100 cc aqueous solution of 12-tungstophosphoric acid (0.1N, containing 28.8g ( $H_3PO_4.12WO_3.xH_2O$) was added with stirring 10g of granular montmorillonite clay (Grade 24, fror Engelhard Corporation). Stirring was maintained for 1 to 2 days at room temperature (20° C). The mixtur was then filtered and the solids were washed with distilled water until the washings no longer showe detectable levels of tungsten, and the final product was dried in vacuo at 40° C. About 8.7g of tunç stophosphoric acid-modified clay was recovered. Tungsten content was 1.0 wt%.

## COMPARATIVE EXAMPLE AA

### Phenol/Acetone Generation using Unmodified Clay Catalyst

To a 250 ml round bottom flask fitted with a condenser heater, stirrer and feed control , was charged mixture of 60.0g of acetone and 0.1g of Engelhard Clay-24. The mixture was heated to reflux (57° C) wit stirring, and 40.0g of "80%" cumene hydroperoxide solution were added dropwise such that the pc temperature did not exceed 66° C.

Small samples (≈2 ml ) of the reactant solution were withdrawn at regular intervals and analyzed by glc The composition of the "80%" cumene hydroperoxide feed was as in Example 1.

The phenol content of the reactant solution is illustrated by the symbol X in Figure 1 of th accompanying drawings. After 1 hour reaction time:

Estimated cumene hydroperoxide conversion: ≈24%

Estimated phenol yield: ≈23 mole %

| Composition of the product solution after 1 hour: | |
|---|---|
| Acetone | 64.4 wt% |
| Cumene | 5.1 wt% |
| Methyl styrene | 0.1 wt% |
| Phenol | 4.5 wt% |
| 2-phenyl-2-propanol | 1.8 wt% |
| Acetophenone | 0.2 wt% |
| Cumene hydroperoxide | 23.8 wt% |

## COMPARATIVE EXAMPLE BB

### Phenol/Acetone Generation Without Catalyst

Following the procedures of Comparative Example AA 40.0g of "80%" cumene hydroperoxide wa diluted with acetone (60.0g) and heated under reflux (66° C) in the absence of any added clay catalyst.

Samples taken after 1 hour show:

Cumene hydroperoxide conversion = <1%
Phenol content = <0.1 wt%
Estimated phenol yield = <0.1%

The results are shown by the symbol ⊙ in Figure 1 of the drawings.

## EXAMPLE 53

### Phenol/Acetone Generation using 12-Tungstophosphoric Acid Modified Clay

To a 250 ml round bottom flask fitted with a condenser heater, stirrer and feed control, was charged mixture of 60.0g of acetone and 0.1g of the 12-tungstophosphoric acid modified clay of Example 52. Th mixture was heated under reflux (57° C) with stirring and 40.0g of the 80% cumene hydroperoxide solutic of comparative Example AA were added dropwise such that the pot temperature did not exceed 66° C.

Small samples (≈2 ml ) of the reactant solution were withdrawn at regular intervals and analyzed by glc

The phenol content of the reactant solution is illustrated in Figure 2. After 30 minutes reaction time:

Estimated cumene hydroperoxide conversion: 96%
Estimated phenol yield: 95 mole %

| Composition of the product solution after 30 minutes: | |
|---|---|
| Acetone | 72.8 wt% |
| Cumene | 5.4 wt% |
| α-Methyl styrene | 0.1 wt% |
| Phenol | 18.5 wt% |
| 2-phenyl-2-propanol | 1.4 wt% |
| Acetophenone | 0.2 wt% |
| Cumene hydroperoxide | 1.3 wt% |

The estimated rate of phenol formation with the tungstophosphoric acid modified clay in this Example at least 6 times faster than with the unmodified clay of comparative Example AA.

EXAMPLES 54 to 56

Phenol/Acetone Generation using other Heteropoly Acid Modified Clay Catalysts

Following the procedures of Example 53, to a 250 ml round bottom flask fitted with condenser, heate stirrer and feed control, was charged with a mixture of 60.0g of acetone and 0.1g of heterpoly acid (1: molybdophosphoric acid, 12-tungstosilicic acid and 12-molybdosilic acid) -modified Clay-24 prepared b the method of Example 52. The mixture was heated to reflux (57° C) with stirring and 40.0g of the 80ᶜ cumene hydroperoxide solution were added dropwise such that the pot temperature did not exceed 68° C.

Small samples (≈2 ml ) of the reactant solution were withdrawn at regular intervals and analyzed by glc

The phenol content of the reactant solutions is illustrated in the accompanying Figures 3 to 5, and th compositions of the product solutions after 1 hour are summarized in Table XIX.

Again, it may be noted that the rate of phenol formation with each of these three heteropoly acic modified Clay-24 catalysts is at least six times faster than with the unmodified Clay-24 of comparativ Example AA.

EXAMPLE 57

Preparation of Tungstophosphoric Acid Modified Clay

To a 2 litre aqueous solution of 12-tungstophosphoric acid (0.01N, containing 57.6g of $H_3 PO_4$ WC $xH_2O$) was added with stirring 200g of extruded montmorillonite clay (Grade 62, from Engelhard, 1.65 mı extrudates). Stirring was maintained for 2 days at room temperature. The extrudates were then recovere by filtration, washed with distilled water until tungsten was no longer detected in the washings, dried ı vacuo at 40° C, and sieved through 10 and 20 mesh (1.68 mm and 0.841 mm) screens.

Tungsten content of the finished extrudates was 0.3 wt%.

EXAMPLE 58

Phenol/Acetone Generation using 12-Tungstophosphoric Acid Modified Clay Catalyst

To a continuous, plug flow reactor equipped with heating/cooling capabilities was charged 150 cc of th 12-tungstophosphoric acid-modified Clay-62 catalyst of Example 57. The catalyst was pretreated with stream of acetone at 60° C, and then 80% cumene hydroperoxide, diluted with an acetone/cumene/phen mixture was passed through the catalyst bed in the upflow mode at a reactor temperature of 60° C and at series of three feed flow rates.

Composition of the feed and product solutions are summarized in Table XX.

In Sample 7, run at a feed LHSV of 10:

Estimated phenol yield: 99 mole %

Estimated acetone yield: >99 mole %

TABLE XIX

| Example | Catalyst[a] | Product Liquid Composition (wt%)[b] | | | | | | | |
|---|---|---|---|---|---|---|---|---|---|
| | | Acetone | Cumene | α-Methyl Styrene | Phenol | 2-Phenyl 2-propanol | Aceto-Phenone | Cumene HydroPeroxide | Yield (Mole %) |
| 54 | $H_3PO_4.12MoO_3$/Clay-24 | 74.0 | 5.2 | 0.3 | 18.7 | 1.0 | 0.2 | 0.1 | 96 |
| 55 | $H_3SiO_4.12MoO_3$/Clay-24 | 73.7 | 4.8 | 0.3 | 19.6 | 1.1 | 0.2 | - | >99 |
| 56 | $H_3SiO_4.12WO_3$/Clay-24 | 74.0 | 4.8 | 0.3 | 19.2 | 1.1 | 0.2 | 0.1 | 99 |

(a)Heteropoly acid on Clay-24 catalyst prepared according to the procedure of Example 52

(b)After 1 hour reaction time

TABLE XX

| Catalyst | Pressure (MPa) | Temp Dow Therm Bed | Feed Rate (Kg/h) | Sample | Acetone | Mesityl Oxide | Cumene | Phenol | Alpha-Methyl Styrene | Aceto-Phenone | 2-Phenyl 2-propanol | Cumene Hydroperoxide | 4-Cumyl Phenol |
|---|---|---|---|---|---|---|---|---|---|---|---|---|---|
| Example 57[a] | 2.17 | 60 | 0.45 | 1 | 34.8 | - | 13.0 | 50.4 | 0.7 | 0.2 | 0.1 | - | 0.4 |
| | | | | 2 | 34.8 | - | 13.0 | 50.5 | 0.7 | 0.2 | 0.1 | - | 0.3 |
| | | | | 3 | 29.7 | - | 13.6 | 54.9 | 0.8 | 0.2 | 0.1 | - | 0.4 |
| | | | 0.75 | 4 | 34.8 | - | 13.0 | 50.4 | 0.7 | 0.2 | 0.1 | - | 0.3 |
| | | | | 5 | 35.0 | - | 13.0 | 50.3 | 0.7 | 0.2 | 0.1 | - | 0.4 |
| | | | | 6 | 34.0 | - | 13.1 | 51.2 | 0.7 | 0.2 | 0.1 | - | 0.3 |
| | | | 1.5 | 7 | 34.7 | - | 13.0 | 50.3 | 0.8 | 0.2 | 0.1 | 0.1 | 0.4 |
| | | | | 8 | 34.8 | - | 13.0 | 50.2 | 0.8 | 0.2 | 0.1 | 0.2 | 0.4 |
| | | | | 9[b] | 35.0 | - | 13.1 | 50.1 | 0.7 | 0.2 | 0.1 | 0.3 | 0.3 |
| Feed: | | | | 1 | 25.8 | - | 13.2 | 37.0 | - | 0.1 | 1.7 | 22.2 | - |
| | | | | 2 | 26.0 | - | 13.2 | 36.6 | - | 0.1 | 1.7 | 22.3 | - |
| | | | | 3 | 26.0 | - | 13.2 | 36.7 | - | 0.1 | 1.7 | 22.2 | - |

(a)Tungstophosphoric acid treated Clay-62.

(b)Recovered catalyst extremely clean, % ash - 87.0

The data in Examples 59 to 67 illustrate:

a) The preparation of zirconium(IV) chloride- and titanium(IV) chloride-modified clays (see Example 59 and 60).

b) Phenol/acetone generation using the zirconium-modified clay catalyst of Example 59 - this gives least a 6-fold improvement in rate in comparison with the unmodified clay (cf. Example 61 and Comparati Example AA).

c) Phenol/acetone generation using the titanium-modified clay catalyst of Example 60 which gives least a 10-fold improvement in rate in comparison with the unmodified clay (cf. Example 62 ar Comparative Example AA).

d) Phenol/acetone generation using the aluminium-modified clay catalyst where the aluminium sourc is Al $(NO_3)_3$ - this catalyst also improves the rate in comparison with the unmodified clay (cf. Example 6 and Comparative Example AA).

e) Phenol/acetone generation using an aluminium(III) chloride-modified clay catalyst - again there an improvement in rate (Example 64).

f) Phenol/acetone generation using a clay catalyst modified with an ethanolic solution of zirconiur (IV) chloride (Example 65)

g) Preparation of a titanium(IV) chloride-modified clay in extrudate form (Example 66).

h) The use of the titanium-modified clay extrudates in the continuous generation of phenol/acetone high space velocities and low operating temperatures (see Example 67).

## EXAMPLE 59

### Preparation of Zirconium(IV) Chloride-Modified Clay

To a 1 litre aqueous solution of zirconium(IV) chloride (0.5N, containing 116g of $ZrCl_4$) was added wi stirring 100 cc of granular montmorillonite clay (Grade 24, from Engelhard Corporation). Stirring wa maintained for 1 to 2 days at room temperature (20 °C).

The mixture was then filtered, the solids were washed with distilled water until the washings no long show detectable levels of zirconium ions, and the final product was dried in vacuo at 40 °C. About 90g zirconium-modified clay was recovered.

Zirconium content: 6.9 wt%.

## EXAMPLE 60

### Preparation of Titanium(IV) Chloride-Modified Clay

To a 100 cc aqueous solution of titanium(IV) chloride (0.5N, containing 9.5g of $TiCl_4$) was added wi stirring 10g of granular montmorillonite clay (Grade 24, from Engelhard Corporation). Stirring was mai tained for 2 to 3 days at room temperature.

The mixture was then filtered, the solids were washed with distilled water until the washings no long showed detectable levels of titanium ions, and the final product was dried in vacuo at 40 °C. About 10.1g titanium-modified clay was recovered.

Titanium content: 5.1 wt%.

## EXAMPLE 61

### Phenol/Acetone Generation using Zirconium-Modified Clay Catalyst

To a 250 ml round bottom flask fitted with a condenser, heater, stirrer and feed control, was charged mixture of 60.0g of acetone and 0.1g of the zirconium-modified clay of Example 59. The mixture wa heated to reflux (57° C) with stirring and 40.0g of the 80% cumene hydroperoxide solution were adde dropwise such that the pot temperature did not exceed 66° C.

Small samples (≈2 ml ) of the reactant solution were withdrawn at regular intervals and analyzed by glc

The phenol content of the reactant solution at various times is illustrated in Figure 6, by the symbol c After 30 minutes reaction time:

Estimated cumene hydroperoxide conversion: 98%
Estimated phenol yield: 93 mole %

| Composition of the product solution after 30 minutes: | |
|---|---|
| Acetone | 73.3 wt% |
| Cumene | 5.9 wt% |
| Methyl styrene | 0.3 wt% |
| Phenol | 18.0 wt% |
| 2-phenyl-2-propanol | 1.3 wt% |
| Acetophenone | 0.2 wt% |
| Cumene hydroperoxide | 0.7 wt% |

A repeat run using a second sample of zirconium-modified clay catalyst is illustrated by the symbol in Figure 6.

Again essentially all the cumene hydroperoxide has reacted in less than 30 minutes reaction time.

The estimated rate of phenol formation with the zirconium-modified clays in this example is at least times faster than with the unmodified clay of Comparative Example AA.

## EXAMPLE 62

### Phenol/Acetone Generation using Titanium-Modified Clay Catalyst

To a 250 ml round bottom flask fitted with a condenser, heater, stirrer and feed control , was charged mixture of 60.0g of acetone and 0.1 g of the titanium-modified clay of Example 60. The mixture was heate to reflux (57° C) with stirring and 40.0g of the 80% cumene hydroperoxide solution was added dropwis such that the pot temperature did not exceed 68° C.

Small samples (≈2 ml ) of the reactant solution were withdrawn at regular intervals and analyzed by glc

The phenol content of the reactant solution is illustrated in Figure 7, by the symbol ⊙. After 20 minute reaction time:

Estimated cumene hydroperoxide conversion: >99%
Estimated phenol yield: 96 mole %.

| Composition of the product solution after 20 minutes: | |
|---|---|
| Acetone | 74.3 wt% |
| Cumene | 5.2 wt% |
| Methyl styrene | 0.6 wt% |
| Phenol | 18.6 wt% |
| 2-phenyl-2-propanol | 0.7 wt% |
| Acetophenone | 0.2 wt% |
| Cumene hydroperoxide | <0.1 wt% |

A repeat run using a second sampl e of titanium-modified clay catalyst is illustrated by the symbol X i Figure 7.

Again essentially all the cumene hydroperoxide had reacted in less than 20 minutes reaction time.

The estimated rate of phenol formation with the titanium-modified clays is at least 10 times faster in thi example than with the unmodified clay of Comparative Example AA.

## EXAMPLE 63

### Phenol/Acetone Generation using Aluminium-Modified Clay Catalyst

An aluminium-modified montmorillonite clay catalyst was prepared by treatment of Engelhard Clay-2· with an aqueous (0.5N) solution of aluminium(III) nitrate following the procedures of Example 59.

A 0.1g sample of this aluminium-modified clay was then utilized as catalyst for the conversion c cumene hydroperoxide (40g) into phenol/acetone following the procedures of Example 61.

After 1 hour reaction time:

Estimated cumene hydroperoxide conversion: 98%

Estimated phenol yield: >95 mole %.

| Composition of the present solution after 1 hour: | |
|---|---|
| Acetone | 70.0 wt% |
| Cumene | 5.9 wt% |
| Methyl styrene | 0.3 wt% |
| Phenol | 22.3 wt% |
| 2-phenyl-2-propanol | 1.4 wt% |
| Acetophenone | 0.2 wt% |
| Cumene hydroperoxide | 0.7 wt% |

The estimated rate of phenol formation with the aluminium-modified clay of this example is about ! times faster than with the unmodified clay of Comparative Example AA.

## EXAMPLE 64

### Phenol/Acetone Generation using Aluminium-Modified Clay Catalyst

An aluminium-modified montmorillonite clay catalyst was prepared by treatment of Engelhard Clay-2·

with an aqueous (0.5N) solution of aluminium(III) chloride following the procedures of Example 59.

0.1g of this aluminium-modified clay is then utilized as catalyst for the conversion of cumene hydroperoxide (40g) into phenol/acetone following the procedures of Example 61.

The phenol content of the reactant solution is illustrated in Figure 8. After 1 hour reaction time:
Estimated cumene hydroperoxide conversion: 92%
Estimated phenol yield: 85 mole %.

| Composition of the product solution after 1 hour: | |
|---|---|
| Acetone | 73.0 wt% |
| Cumene | 6.0 wt% |
| Methyl styrene | 0.1 wt% |
| Phenol | 16.5 wt% |
| 2-phenyl-2-propanol | 1.6 wt% |
| Acetophenone | 0.2 wt% |
| Cumene hydroperoxide | 2.4 wt% |

## EXAMPLE 65

### Phenol/Acetone Generation using Zirconium-Modified Clay Catalyst

A zirconium-modified montmorillonite clay catalyst is prepared by treatment of Engelhard Clay-24 with an ethanolic (0.5N) solution of zirconium(IV) chloride following the procedures of Example 60.

The zirconium content of the modified clay was 3.8 wt%.

0.1g of this zirconium-modified clay was then utilized as catalyst for the conversion of cumene hydroperoxide (40g) into phenol/acetone following the procedures of Example 61.

After 1 hour reaction time:
Estimated cumene hydroperoxide conversion: 68%
Estimated phenol yield: 68 mole %.

| Composition of the product solution after 1 hour: | |
|---|---|
| Acetone | 71.3 wt% |
| Cumene | 2.1 wt% |
| Methyl styrene | 0.4 wt% |
| Phenol | 13.3 wt% |
| 2-phenyl-2-propanol | 2.3 wt% |
| Acetophenone | 0.5 wt% |
| Cumene hydroperoxide | 10.1 wt% |

## EXAMPLE 66

### Preparation of Titanium(IV) Chloride-Modified Clay

To a 2 litre aqueous solution of titanium(IV) chloride (0.5N, containing 190g of $TiCl_4$) was added wit stirring 200g of extruded montmorillonite clay (Grade 62, from Engelhard, 1.65 mm extrudates). Stirring wa maintained for 2 days at room temperature.

The extrudates were then recovered by filtration, washed with distilled water, dried in vacuo at 40° C and sieved through 10 and 20 mesh (1.68 mm and 841 um) screens.

Titanium content of the finished extrudates: 2.1 wt%.

## EXAMPLE 67

### Phenol/Acetone Generation using Titanium-Modified Clay Catalyst

To a continuous, plug flow reactor equipped with heating/cooling capabilities was charged 150 cc of th titanium-modified catalyst of Example 66. The catalyst was pretreated with a stream of acetone at 60° C and then 80% cumene hydroperoxide diluted with an acetone/cumene/phenol mix was passed through th catalyst bed in the up flow mode.

A typical liquid feed composition was:

| | |
|---|---|
| Acetone | 25.3% |
| Cumene | 13.4% |
| Phenol | 37.3% |
| α-Methyl styrene | - |
| Acetophenone | 0.1% |
| 2-phenyl-2-propanol | 1.6% |
| Cumene hydroperoxide | 22.2% |

Reactor operating conditions were 60° C, 2.17 MPa pressure and a total liquid feed rate of 1.5 Kg/h, .e. LHSV = 10.

The typical product effluent under these conditions comprises:

| | |
|---|---|
| Acetone | 33.8% |
| Cumene | 13.4% |
| Phenol | 50.6% |
| α-Methyl styrene | 0.6% |
| Acetophenone | 0.2% |
| 2-phenyl-2-propanol | 0.2% |
| Cumene hydroperoxide | 0.2% |

Estimated cumene hydroperoxide conversion: >99%

Estimated yield of phenol : 97 mole %

Estimated yield of acetone: >99 mole %

The data in Examples 68 to 71 illustrate:

1. Regeneration of Engelhard Clay, Grade 24, used in phenol/acetone generation, by Soxhl extraction with concentrated nitric acid. The regenerated catalyst (6337-59-1) showed the following i comparison with a control sample of used catalyst, (6337-49-1-1), Example 68:

a) Improved phenol productivity - see Figure 9.

b) Higher levels of cumene hydroperoxide conversion.

c) Higher ash contents - and therefore a lower level of organic contaminants.

d) Improved colour - pale yellow versus yellow-brown-indicative of reduced levels of organic polym (phenolic resins, etc.).

This regenerated catalyst also showed higher activity in comparison with fresh samples of Clay-24, se Figure 9.

2. Regeneration of Engelhard Clay, Grade 25, used in phenol/acetone service by Soxhlet extractic with concentrated nitric acid. The regenerated catalyst (6337-96-1-5) again shows (in comparison with control sample of used catalyst, (6337-84-1-5)), Example 69:

a) Improved phenol productivity - see Figures 10 and 11.
b) Higher ash levels of cumene hydroperoxide conversion.
c) Higher ash contents.
d) Improved colour.

3. Regeneration of Engelhard Clay, Grade 25, used in phenol/acetone service by treatment wi methanol, particularly at higher temperatures. Once again the regenerated catalysts show as demonstrate by Examples 70 and 71:

a) Improved phenol productivity - see Figure 10.
b) Higher levels of cumene hydroperoxide conversion.
c) High ash contents.

## EXAMPLE 68

A 150 cc sample of Engelhard Clay-24 was subjected to 3 days of use as a catalyst for phenol/acetor generation from cumene hydroperoxide. The recovered catalyst comprised brown and yellow granule sample 6337-49-1-1.

An analysis for ash content of the fresh and used Clay-24 gave the following results.

| | |
|---|---|
| Fresh Clay | 80.7% |
| Used Clay-24 | 68.3% |

An activity test for samples of the new and used Clay-24 catalyst was also conducted as follows:

To a 250 cc round bottom flask fitted with a condenser, heater, stirrer and feed control, was charged mixture of 60.0g of acetone and 0.1 g of Clay-24 catalyst. The mixture was heated to reflux (57°C) wit stirring, and 40.0g of 80% cumene hydroperoxide solution were added dropwise such that the p temperature did not exceed 66°C. Small samples of the reactant solution (≈2 cc) were withdrawn at regul intervals and analyzed by glc.

The phenol content of the reactant solutions using the new and used Clay-24 catalyst is illustrated Figure 9 by the symbols X and ⊙ respectively. After 1 hour reaction time:

For the fresh Clay-24 catalyst:
Estimated cumene hydroperoxide conversion: 24%
Estimated phenol yield: 23 mole %
Whereas for the used Clay-24 catalyst 6337-49-1-1:
Estimated cumene hydroperoxide conversion: 20%
Estimated phenol yield: 20 mole %

A sample of the used Clay-24 catalyst was then regenerated as described in the following method:

About 20 ml (16.0g) of used Clay-24 (Sample 6337-49-1-1) was placed in a porous ceramic thimble in Soxhlet extractor and extracted with 600 ml of refluxing concentrated nitric acid for a period of 3 hour After cooling, the solid catalyst was washed with copious amounts of water and acetone, and dried in vacu 13.5 g of pale yellow granules were recovered.

An analysis of ash content of the regenerated Clay-24 (Sample 6337-59-1) gave the following result 81.6%.

An activity test for the same regenerated Clay-24, conducted as described above, is also shown Figure 9 by the symbols △. After 1 hour reaction time:

Estimated cumene hydroperoxide conversion: 50%
Estimated phenol yield: 40 mole %.

## EXAMPLE 69

A 150 cc sample of Engelhard clay, Grade 25, was subjected to 3 days of use as a catalyst fc phenol/acetone generation from cumene hydroperoxide. The recovered catalyst comprised brown an yellow granules, Sample 6337-84-1-5.

An analysis for ash content of the used Clay-25 gave the following result - 71.8%.

An activity test for the same sample was also completed as described in Example 68. The phen content of the reactant solution is illustrated in Figure 10 △ . After 1 hour reaction time (Run 6337-93):
Estimated cumene hydroperoxide conversion: 19%
Estimated phenol yield: 13 mole %

A sample (20 cc) of the used clay-25 catalyst was then regenerated by extraction with concentrate nitric acid as described in Example 68. After cooling, the solid catalystg was washed with water an acetone, and dried in vacuo. 12. 6g of pale yellow granules were recovered.

An analysis of the ash content of the regenerated Clay-25 (Sample 6337-96-1-5) gave the followin result -90.6%.

An activity test for the same regenerated Clay-25, conducted as described in Example 68, is shown i Figure 11. After 1 hour reaction time:
Estimated cumene hydroperoxide conversion: 99%
Estimated phenol yield: 96 mole %

## EXAMPLE 70

A sample of the used Engelhard Clay Grade 25, catalyst of Example 69 was also regenerated b treatment with methanol as described in the following method:

About 30g of used Clay-25 (Sample 6337-84-1-5) was placed in a round bottom flask fitted with condenser, and refluxed with 100 cc of methanol for 6 hours. On cooling the remaining solids wer recovered by filtration, washed with methanol and dried in vacuo.

An analysis of the ash content of the regenerated Clay-25 (Sample 6337-84-1-reg) gave the followin result - 92.5%.

An activity test for the same regenerated Clay-25, conducted as described in Example 68, is shown i Figure 10 (symbol ⊙). After 1 hour reaction time:
Estimated cumene hydroperoxide conversion: 23%
Estimated phenol yield: 15 mole %

## EXAMPLE 71

Another sample of the used Engelhard Clay Grade 25, catalyst of Example 69 was regenerated b treatment with methanol, but this time at higher temperatures in an autoclave, as described in the followin method:

About 30g of used Clay-25 (Sample 6337-84-1-5) was placed in a 300 cc stainless steel autoclave wit 100 cc of methanol, and the mixture was heated to 150° C, with rocking, for 6 hours. On cooling, th remaining solids were recovered by filtration, washed with methanol, and dried in vacuo.

An analysis of the ash content of the regenerated Clay-25 (Sample 6337-84-2-reg) gave the followin result - 91.7%.

An activity test for the same regenerated Clay-25, conducted as described in Example 68, is shown i Figure 10 (symbol X) . After 1 hour reaction time:
Estimated cumene hydroperoxide conversion: 41%
Estimated phenol yield: 32 mole %.

## Claims

1. A method for the production of phenol and acetone by the acid-catalyzed decomposition of cumen hydroperoxide, characterized in that cumene hydroperoxide is decomposed at a temperature of 20 t 150° C and a pressure of 0.1 to 7 MPa in the presence of a heterogeneous catalyst selected from:
a heteropoly acid on an inert support,

an ion exchange resin with a sulfonic acid functionality,
an acidic smectite clay,
a fluorine-containing acid on an inert support, and montmorillonite clay modified by a heteropoly acid or I titanium, zirconium or aluminium.

2. A method according to Claim 1 characterized in that the heteropoly acid catalyst is a support heteropoly acid having the Keggin structure

$H_{8-n}[XM_{12}O_{40}]$

in which X is P or Si , M is Mo or W, and n is 4 or 5.

3. A method according to Claim 1 characterized in that the ion exchange resin catalyst is an i exchange resin having sulphonic acid functionality bonded directly or indirectly to a backbone of polyst rene or styrene -divinylbenzene polymer having 1 to 20% crosslinking.

4. A method according to Claim 1 characterized in that the acidic smectite clay catalyst is montmorillonite silica-alumina clay having the structure

$M_{x/n}$ - y $H_2O(Al_{4-x}Mg_x)(Si_8)O_{20}(OH)_4$

in which M is sodium or lithium, x, y and n are numbers, and the clay has an acidity of at least 15 n KOH/g.

5. A method according to Claim 1 characterized in that the fluorine-containing acid catalyst compris hydrofluoric acid or a fluorophosphoric acid on a titanium dioxide, alumina or silica support.

6. A method according to Claim 1 characterized in that the modified montmorillonite clay is a modifie clay of the structure

$M_{x/n}$ - y $H_2O(Al_{4-x}Mg_x)$ $(Si_8)O_{20}(OH)_4$

modified with a heteropoly acid having the Keggin structure

$H_{8-n}[XM_{12}O_{40}]$

wherein X is P or Si , M is Mo or W, and n is 4 or 5, or with an inorganic salt of titanium, zirconium aluminium.

7. A method according to Claim 6 characterized in that, after use, the catalyst is regenerated I treatgment with nitric acid or methanol.

8. A method according to any one of Claims 1 to 7 characterized in that the temperature is 40 120 °C.

9. A method according to any one of Claims 1 to 8 characterized in that the pressure is 0.8 to 2.ℓ MPa.

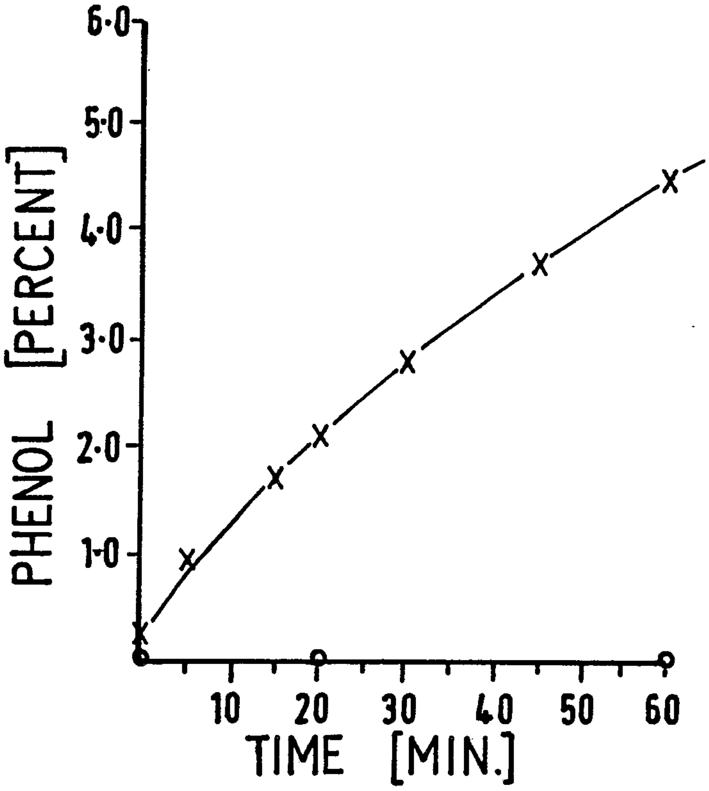


FIG.1

FIG.2

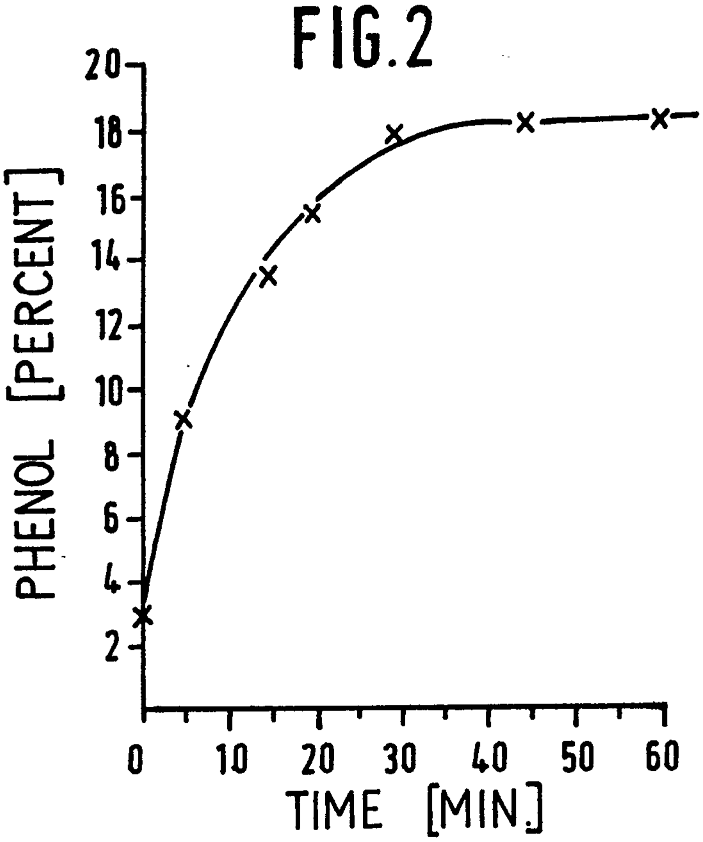
PHENOL [PERCENT]
20
18
16
14
12
10
8
6
4
2
0
10
20
30
40
50
60
TIME [MIN.]

FIG.3

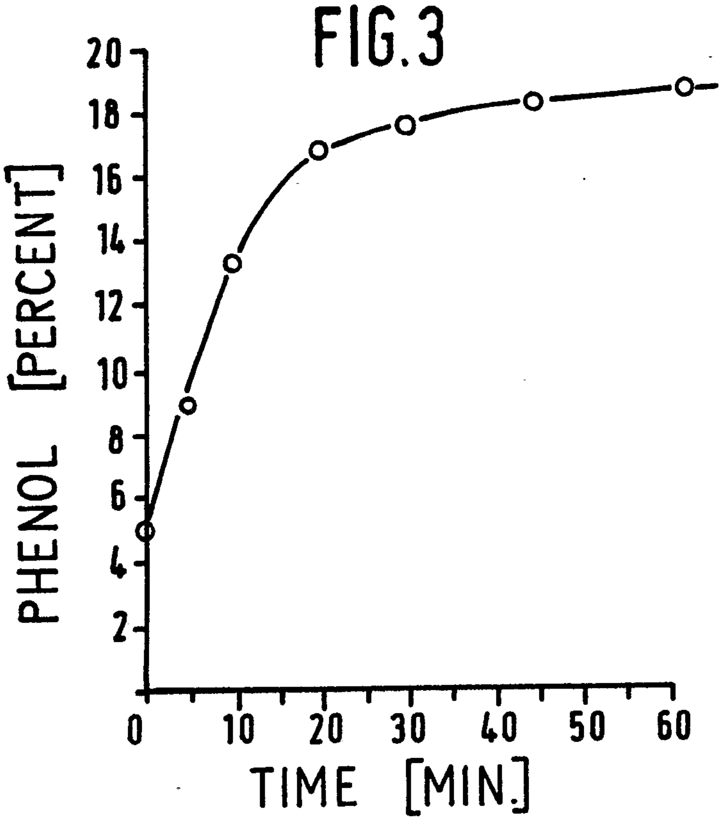
PHENOL [PERCENT]
20
18
16
14
12
10
8
6
4
2
0
10
20
30
40
50
60
TIME [MIN.]

FIG.4

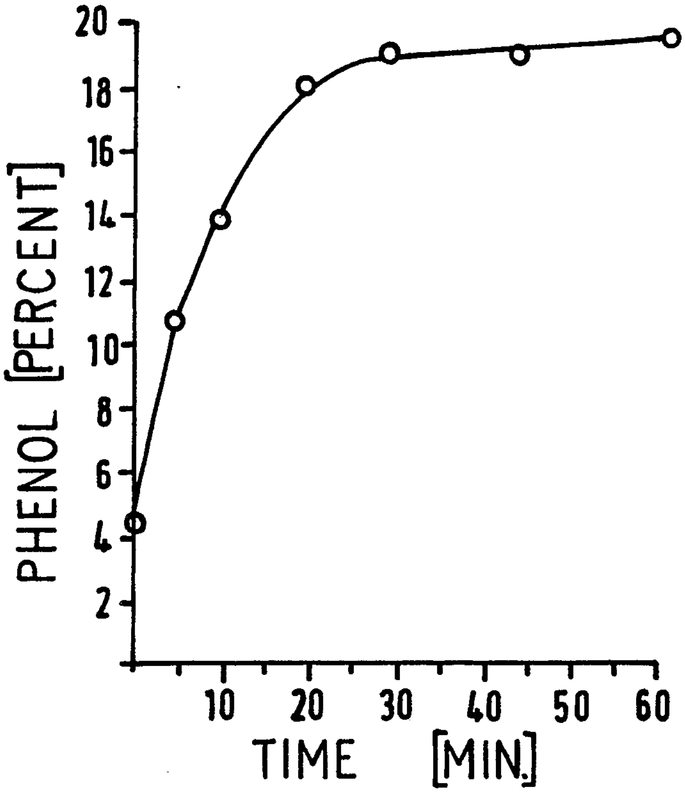
20
18
16
14
12
10
8
6
4
2
PHENOL [PERCENT]
10
20
30
40
50
60
TIME [MIN]

FIG.5

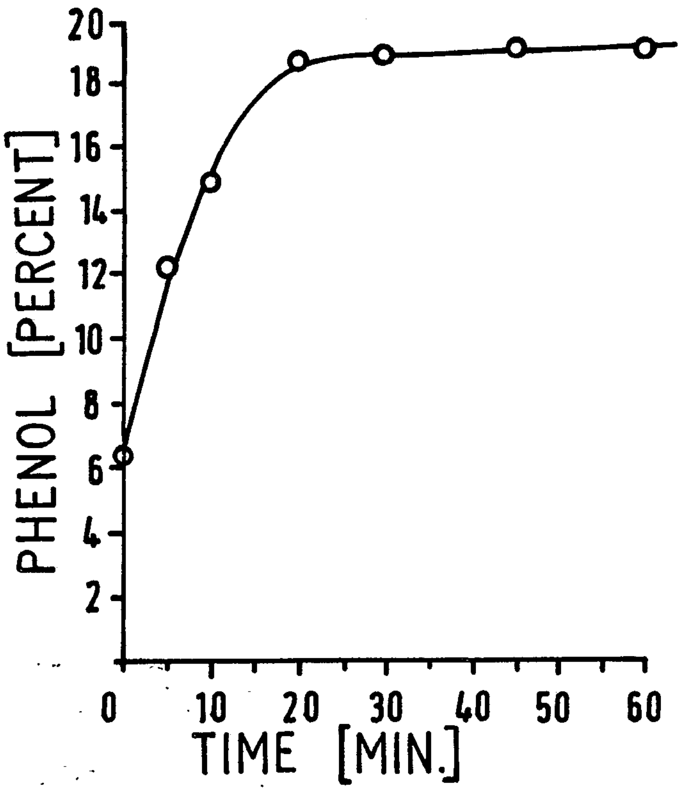
20
18
16
14
12
10
8
6
4
2
PHENOL [PERCENT]
0
10
20
30
40
50
60
TIME [MIN.]

# FIG. 6

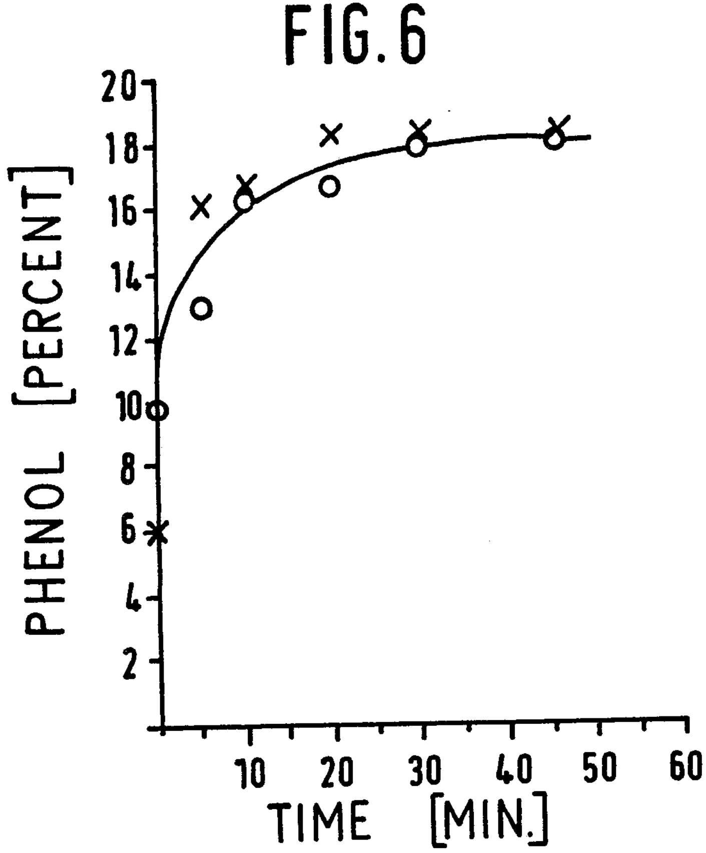

PHENOL [PERCENT]
20
18
16
14
12
10
8
6
4
2
10
20
30
40
50
60
TIME [MIN.]


# FIG. 7

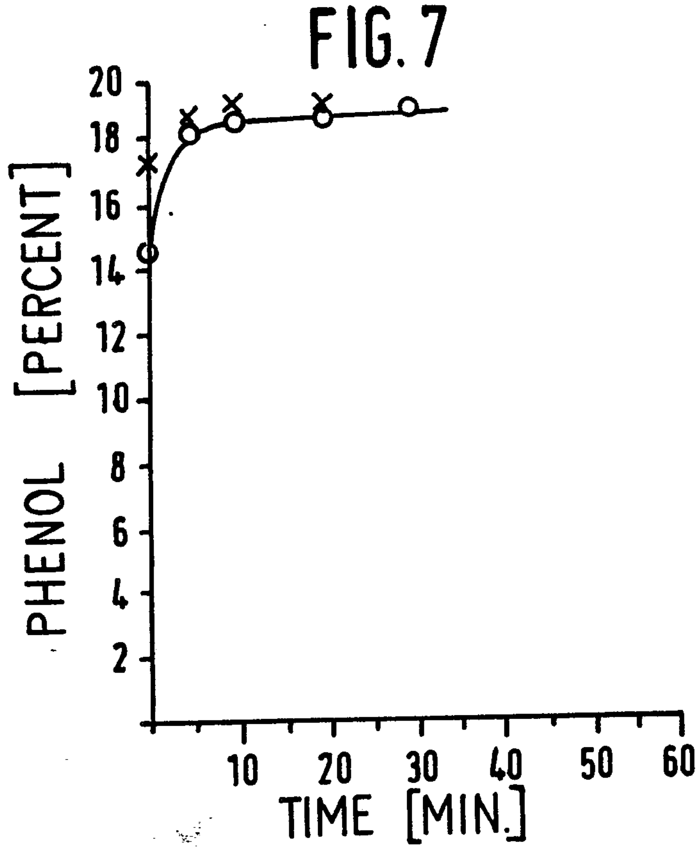

PHENOL [PERCENT]
20
18
16
14
12
10
8
6
4
2
10
20
30
40
50
60
TIME [MIN.]

FIG. 8

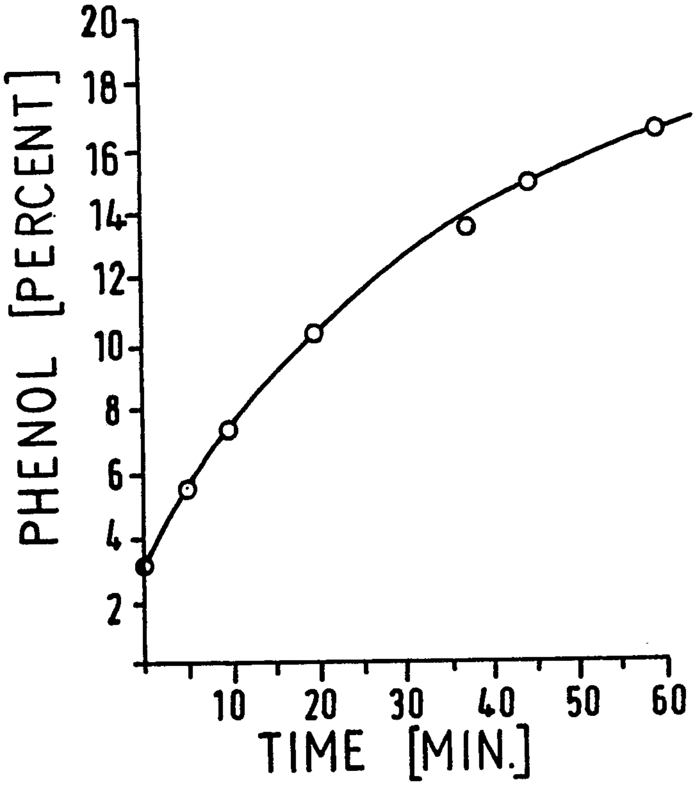
PHENOL [PERCENT]
20
18
16
14
12
10
8
6
4
2
10
20
30
40
50
60
TIME [MIN.]

FIG. 9

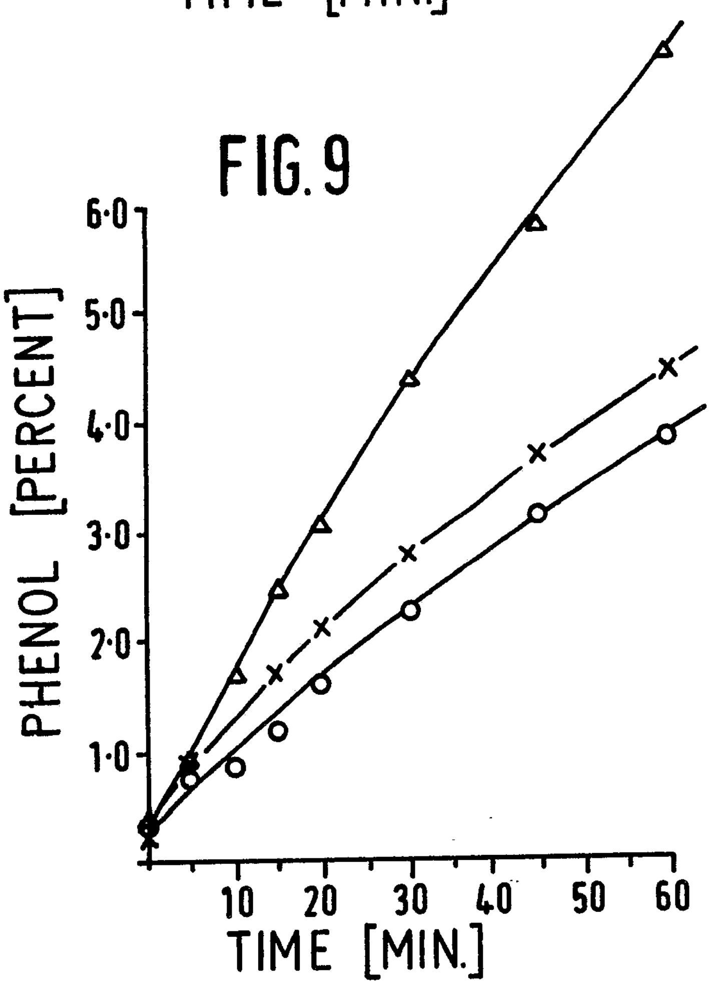
PHENOL [PERCENT]
6·0
5·0
4·0
3·0
2·0
1·0
10
20
30
40
50
60
TIME [MIN.]

FIG. 10

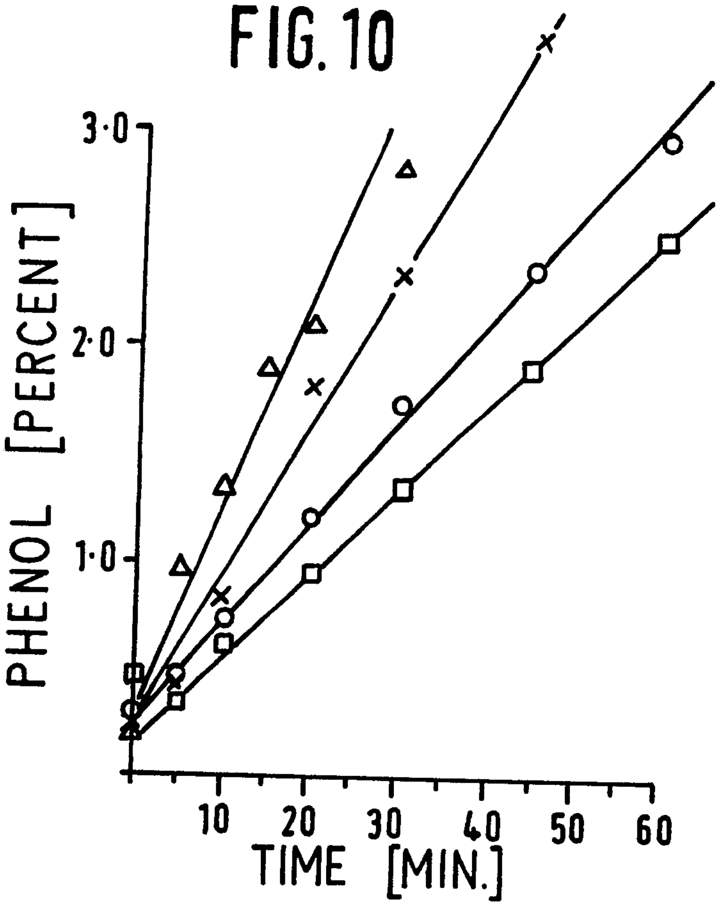
PHENOL [PERCENT]
3·0
2·0
1·0
10
20
30
40
50
60
TIME [MIN.]

FIG. 11

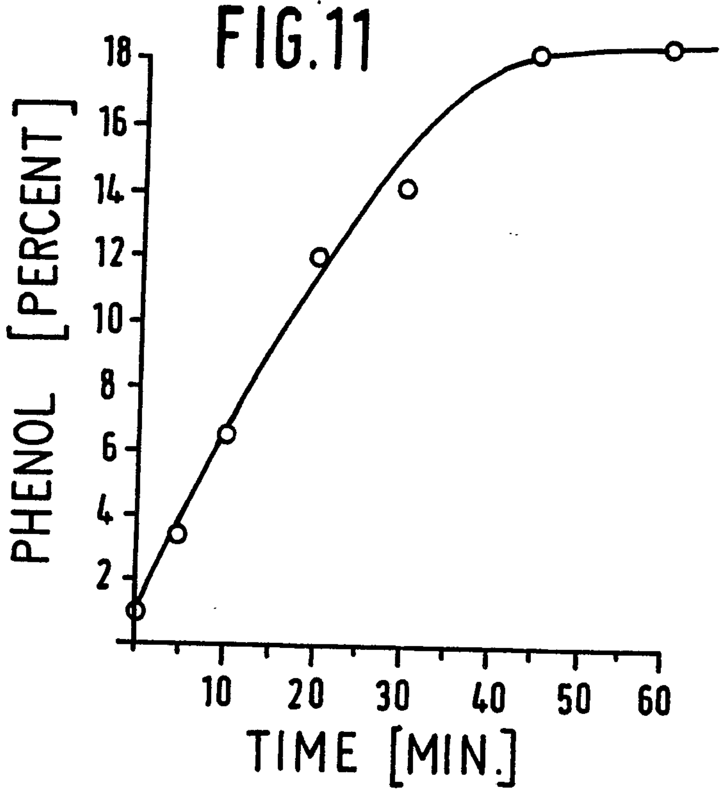
PHENOL [PERCENT]
18
16
14
12
10
8
6
4
2
10
20
30
40
50
60
TIME [MIN.]